# EUROPEAN PATENT SPECIFICATION

(11) **EP 3 889 587 B1**
(45) Date of publication and mention of the grant of the patent: **11.03.2026**
(21) Application number: 19903711.0
(22) Date of filing: 11.12.2019
(51) Int. Cl.: G01N 21/78, G01N 21/84, G01N 21/77

(54) **TEST STRIP AND COMPONENT MEASUREMENT SYSTEM**
TESTSTREIFEN UND BAUTEILMESSSYSTEM
BANDELETTE DE TEST ET SYSTÈME DE MESURE DE COMPOSANT

(30) Priority: 28.12.2018 JP 2018247095
(43) Date of publication of application: 06.10.2021
(73) Proprietor: TERUMO Kabushiki Kaisha, Tokyo 151-0072 (JP)
(72) Inventor: MORIUCHI, Takeyuki, Nakakoma-gun, Yamanashi 409-3853 (JP); AIKAWA, Ryokei, Ashigarakami-gun, Kanagawa 259-0151 (JP); SATO, Hiroya, Nakakoma-gun, Yamanashi 409-3853 (JP); HAYASHIDA, Yuma, Nakakoma-gun, Yamanashi 409-3853 (JP)
(74) Representative: Casalonga
(86) International application number: PCT/JP2019/048369
(87) International publication number: WO 2020/137532

(56) References cited:
- EP-A1- 2 781 919
- WO-A1-2016/152225
- WO-A1-2017/154270
- WO-A1-2017/199510
- WO-A1-2018/173609
- JP-A- 2014 190 926
- JP-A- 2016 514 830
- US-A- 5 780 304
- US-A1- 2004 028 557
- US-A1- 2015 185 159

## Description

### Technical Field

The present disclosure relates to a test strip that measures an analyte in a sample, and a component measurement system to which the test strip is attached and that optically measures the analyte. In particular, the present invention relates to a test strip according to the preamble of independent claim 1, such as it is e.g. known from US 2015/ 185159 A1 or US 5 780 304 A.

### Background Art

JP-A-2004-317487 discloses a colorimetric blood glucose monitoring system (component measurement system) that measures an amount of a glucose component in a sample by outputting light from an LED to the sample (blood) and receiving the light transmitted through the sample by a detector. The light from the LED is narrowed by a collimating opening in a component measurement device, and irradiates a reading region (a measurement spot) of the sample.

### Summary of Invention

In this type of component measurement system, even if the light of a light source is narrowed by the opening of the component measurement device, a deviation from the reading region of the sample occurs, so that there is a limit for improvement in measurement accuracy of a blood glucose level.

The disclosure is made to solve the above problem, and an object of the disclosure is to provide a test strip and a component measurement system capable of accurately guiding measurement light of a consistent light amount to a measurement spot with a simple configuration and performing component measurement with higher accuracy.

In order to achieve the above object the present invention provides a test strip according to independent claim 1 as well as a component measurement system according to claim 12. The dependent claims relate to advantageous embodiments.

In the test strip and the component measurement system described above, the light shielding portion of the main body portion defines the measurement spot on the reaction product, and thus the measurement light of a consistent light amount can be accurately guided to the measurement spot. For example, even when the test strip is attached to the component measurement device in a position-deviated manner, it is possible to accurately guide a sufficient amount of the measurement light to the measurement spot of the test strip with reproducibility. Accordingly, the test strip and the component measurement system can perform more accurate component measurement.

### Brief Description of Drawings

[Fig. 1] Fig. 1 is a plan view illustrating an overall configuration of a component measurement system according to an embodiment of the disclosure.
[Fig. 2] Fig. 2 is a perspective view illustrating an overall configuration of a test strip.
[Fig. 3] Fig. 3 is an exploded perspective view of the test strip.
[Fig. 4] Fig. 4 is a schematic side cross-sectional view partially illustrating the test strip and a light measurement unit of a component measurement device.
[Fig. 5] Fig. 5A is an explanatory diagram illustrating a positional relationship between a measurement opening, and an emitting-side passage opening and a light receiving-side passage opening. Fig. 5B is an explanatory diagram illustrating a positional relationship between the measurement opening, and the emitting-side passage opening and the light receiving-side passage opening when the test strip is position-deviated.
[Fig. 6] Fig. 6 is an explanatory diagram schematically illustrating a measurement opening according to a first modification.
[Fig. 7] Fig. 7A is an explanatory diagram schematically illustrating a measurement opening according to a second modification. Fig. 7B is an explanatory diagram schematically illustrating a measurement opening according to a third modification.
[Fig. 8] Fig. 8 is a perspective view illustrating an overall configuration of a test strip according to a modification.
[Fig. 9] Fig. 9 is an exploded perspective view of the test strip in Fig. 8.
[Fig. 10] Fig. 10 is a longitudinal cross-sectional view of the test strip in Fig. 8 (a cross-sectional view taken along an X direction at a center in a Y direction).
[Fig. 11] Fig. 11 is a plan view of the test strip in Fig. 8 as viewed from one side in a thickness direction of the test strip.
[Fig. 12] Fig. 12 is a schematic side cross-sectional view (a cross-sectional view taken along the X direction at the center in the Y direction) partially illustrating the test strip in Fig. 8 and a component measurement device according to a modification.
[Fig. 13] Fig. 13 is a cross-sectional view taken along a line XIII-XIII in Fig. 12.
[Fig. 14] Fig. 14 is an electric block diagram of the component measurement device in Fig. 13.
[Fig. 15] Fig. 15 is a table illustrating Examples 1 to 7 and Comparative Examples 1 to 5.

### Description of Embodiments

Hereinafter, a preferred embodiment of the disclosure will be described in detail with reference to the accompanying drawings.

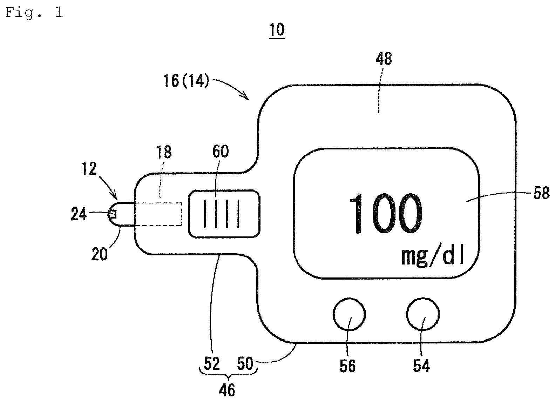

As illustrated in Fig. 1, a component measurement system 10 according to an embodiment of the disclosure includes a test strip 12 capable of holding a sample, and a component measurement device 14 that measures an analyte amount contained in the sample by attaching the test strip 12. Hereinafter, the component measurement system 10 (blood glucose level measurement system) that detects the amount of analyte (here, glucose) when a sample is blood will be representatively described.

In this case, the test strip 12 introduces blood into the test strip 12. The test strip 12 is configured to hold the blood and a reagent at a detection target position in a state in which the blood and the reagent are reacted with each other and colored (coloration state). On the other hand, the component measurement device 14 is configured as a blood glucose meter 16 (hereinafter, also referred to as the blood glucose meter 16) that optically detects a reaction product of the blood and the reagent at the detection target position of the test strip 12. Particularly, the blood glucose meter 16 performs blood glucose level measurement by a measurement unit 18 that irradiates the detection target position with measurement light of a predetermined wavelength and detects the measurement light (transmitted light) that passes through the blood in the coloration state. The test strip may also be referred to as a chip, a sensor, or the like.

The component measurement system 10 is used as a measurement system for personal use operated by a user (a patient). For example, the user uses the test strip 12 and the blood glucose meter 16 to measure the blood glucose level and manage his/her own blood glucose. The component measurement system 10 may be used in a medical facility or the like as a device for a medical worker to measure the blood glucose level of the patient.

When the test strip 12 is attached to the blood glucose meter 16, a part of the test strip 12 protrudes to an outside of the blood glucose meter 16. Further, the test strip 12 has an opening (an intake portion 24) at a portion protruding from the blood glucose meter 16. By introducing the blood into the test strip 12 from the intake portion 24, the blood glucose level can be measured by the blood glucose meter 16. The test strip 12 is a disposable device that is discarded after each measurement.

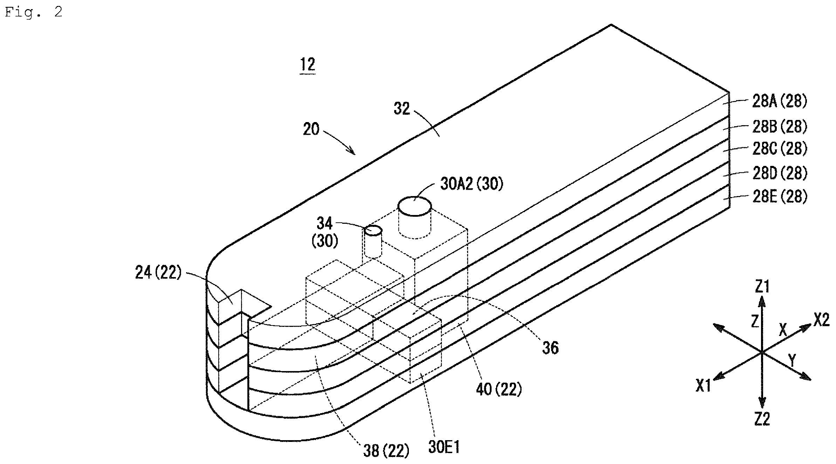

As illustrated in Fig. 2, the test strip 12 includes a test paper-shaped main body portion 20 and a cavity portion 22 extending in a longitudinal direction (an arrow X direction) along a planar direction in the main body portion 20. The cavity portion 22 has a flow path 38 (capillary) for introducing the sample from the intake portion 24 to a measurement spot 44 to be described later, and an air vent portion 40 to be described later. In the main body portion 20, insertion and removal directions of the blood glucose meter 16 are a major axis direction of the main body portion 20. Here, when the main body portion 20 is attached to the blood glucose meter 16, an end on a side exposed from the blood glucose meter 16 (an end in an arrow X1 direction) is referred to as a first end, and an end on a side accommodated in the blood glucose meter 16 (an end in an arrow X2 direction) is referred to as a second end. The first end side of the main body portion 20 is formed in a substantially semicircular shape in a plan view. The first end side of the main body portion 20 is a portion that is exposed from the blood glucose meter 16 in an attached state to the blood glucose meter 16.

A first end of the cavity portion 22 (an end in the arrow X1 direction) is opened at an outer edge of the first end of the main body portion 20.

The first end of the cavity portion 22 is formed with the intake portion 24 as an opening for taking the blood into the main body portion 20.

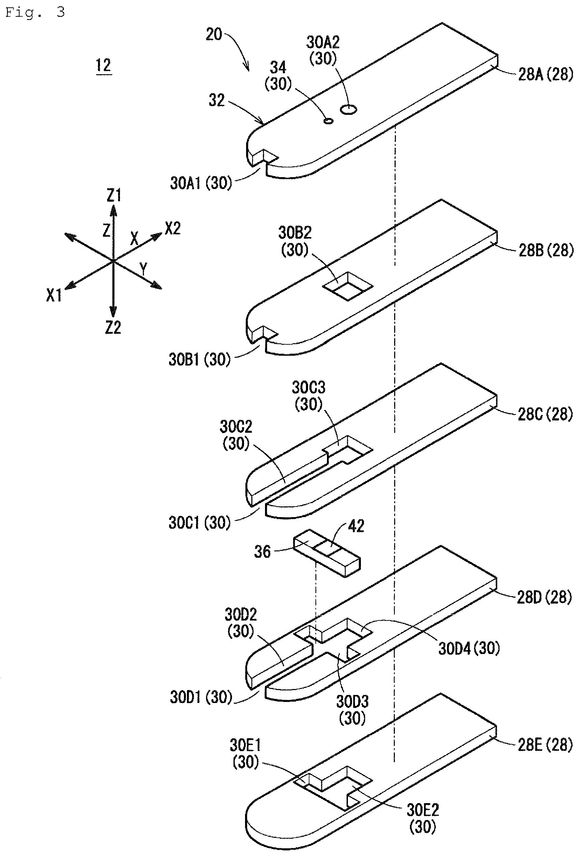

The main body portion 20 is formed in a thin flat plate shape (test paper shape). More specifically, as illustrated in Figs. 2 and 3, the main body portion 20 is formed by stacking and integrating a plurality of plate bodies 28 in a thickness direction (an arrow Z direction) of the plate bodies 28. Hereinafter, the plurality of plate bodies 28 are referred to as a first plate body 28A, a second plate body 28B, a third plate body 28C, a fourth plate body 28D, and a fifth plate body 28E from an upper direction (an arrow Z1 direction) toward a lower direction (an arrow Z2 direction) in Fig. 3. Outer edges of the first to fifth plate bodies 28A to 28E are formed in substantially the same shape (a substantially rectangular shape having an arc on the first end side) in a plan view from the arrow Z direction.

Each of the first to fifth plate bodies 28A to 28E is cut out before being stacked, so that the first to fifth plate bodies 28A to 28E each has a partial space 30 that penetrates in the thickness direction (the arrow Z direction) and is formed in an appropriate shape. In the partial space 30, the cavity portion 22, a measurement opening 34 (aperture), and the air vent portion 40 of the test strip 12 are formed in a state in which the first to fifth plate bodies 28A to 28E are stacked. An adhesive layer (not illustrated) made of an adhesive or the like is provided between the plate bodies 28 adjacent to each other. The adjacent plate bodies 28 are firmly adhered to each other by the adhesive layer.

Specifically, the first plate body 28A is a planar member disposed on one side in the thickness direction of the test strip 12 (the end in the arrow Z1 direction). The first plate body 28A is constituted as a black film member, and includes a light shielding portion 32 that shields the measurement light in the test strip 12. A configuration of the light shielding portion 32 will be described in detail later.

The partial space 30 of the first plate body 28A includes a first cutout portion 30A1 provided on the first end side (the end in the arrow X1 direction), the measurement opening 34, and a first space portion 30A2.

The first cutout portion 30A1 forms a part of the intake portion 24. The first cutout portion 30A1 is formed in a quadrangular shape (a rectangular shape in the illustrated example) in a plan view as viewed from the arrow Z direction. The measurement opening 34 is closer to the first end side than the first space portion 30A2. The measurement opening 34 is independently provided at a position separated from the first cutout portion 30A1 by a predetermined distance toward the second end side of the first plate body 28A (the arrow X2 direction). The measurement opening 34 transmits the measurement light in the thickness direction of the test strip 12. The first plate body 28A may be manufactured of a transparent member only in a range corresponding to the measurement opening 34.

The first space portion 30A2 is opened to a surface of the test strip 12 (a surface in the arrow Z1 direction). The first space portion 30A2 is independently provided at a position slightly separated from the measurement opening 34 toward the second end side. When the blood is introduced into the test strip 12 from the intake portion 24, the first space portion 30A2 discharges air pushed out from the cavity portion 22 (the flow path 38 or the like) to the outside of the test strip 12.

The second plate body 28B is stacked on the first plate body 28A on the other side (the arrow Z2 direction) in the thickness direction of the test strip 12. The second plate body 28B is a film-shaped member. The partial space 30 of the second plate body 28B includes a second cutout portion 30B1 on the first end side, and a second space portion 30B2 closer to the first end side of the second plate body 28B and on an inner side in a width direction (an arrow Y direction). The second plate body 28B may be transparent or colored.

The second cutout portion 30B1 forms a part of the intake portion 24. The second cutout portion 30B1 is formed in a quadrangular shape (a rectangular shape in the illustrated example) in a plan view as viewed from the arrow Z direction. In the stacked state, the second cutout portion 30B1 communicates with the first cutout portion 30A1 in the arrow Z2 direction. The second cutout portion 30B1 has the same size and shape as the first cutout portion 30A1.

The second space portion 30B2 is formed at a position facing the first space portion 30A2. That is, the second space portion 30B2 communicates with the first space portion 30A2 in the arrow Z2 direction. The second space portion 30B2 is formed in a rectangular shape having a size larger than that of the first space portion 30A2. The second space portion 30B2 is a space capable of holding a certain amount of blood on the second end side of the flow path 38. Therefore, when the blood leaks from the second end side of the flow path 38, the second space 30B2 functions as a buffer region, and thereby the second space portion 30B2 can prevent the blood from leaking from the first space portion 30A2.

On the other hand, in the second plate body 28B, the partial space 30 is not provided at a portion facing the measurement opening 34 of the first plate body 28A. A portion of the second plate body 28B facing the measurement opening 34 is formed of a transparent member. Accordingly, the second plate body 28B forms a liquid-tight barrier between the measurement opening 34 and the partial space 30 of the third plate body 28C to be described below.

The third plate body 28C is a film-shaped member stacked on the second plate body 28B in the arrow Z2 direction. Upper and lower surfaces of the third plate body 28C have adhesive layers. The partial space 30 of the third plate body 28C includes a third cutout portion 30C1, a first straight line portion 30C2, and a third space portion 30C3. The third cutout portion 30C1, the first straight line portion 30C2, and the third space portion 30C3 are formed in the third plate body 28C as spaces that are continuous from the first end toward the second end side.

The third cutout portion 30C1 forms a part of the intake portion 24. In the stacked state, the third cutout portion 30C1 communicates with the first cutout portion 30A1 and the second cutout portion 30B1 in the arrow Z2 direction (stacked to form one space). The first straight line portion 30C2 communicates with the third cutout portion 30C1 and extends toward the second end side (the arrow X2 direction). The first straight line portion 30C2 is a space constituting a part of the flow path (capillary) 38 through which the blood taken in from the intake portion 24 flows.

The third space portion 30C3 is continuous with the first straight line portion 30C2. The third space portion 30C3 faces the second space portion 30B2 in the thickness direction of the test strip 12 (the arrow Z direction). In the stacked state, the third space portion 30C3 forms a continuous space together with the second space portion 30B2, and communicates with the outside of the test strip 12 via the first space portion 30A2. The second space portion 30B2 and the third space portion 30C3 are formed in substantially the same shape.

The fourth plate body 28D is stacked on the third plate body 28C in the arrow Z2 direction. The partial space 30 of the fourth plate body 28D includes a fourth cutout portion 30D1, a second straight line portion 30D2, a reagent placement portion 30D3, and a fourth space portion 30D4.

The fourth cutout portion 30D1, the second straight line portion 30D2, the reagent placement portion 30D3, and the fourth space portion 30D4 are connected from the first end toward the second end side to form a continuous space. The fourth cutout portion 30D1 is formed at the first end of the partial space 30, and forms a part of the intake portion 24. The fourth cutout portion 30D1 communicates with the third cutout portion 30C1 in the arrow Z2 direction. The second straight line portion 30D2 communicates with the fourth cutout portion 30D1 and extends toward the second end side (the arrow X2 direction). The second straight line portion 30D2 is a space constituting a part of the flow path (capillary) 38 through which the blood taken in from the intake portion 24 flows.

The fourth space portion 30D4 communicates with the second straight line portion 30D2 via the reagent placement portion 30D3. The fourth space portion 30D4 faces the third space portion 30C3 in the thickness direction of the test strip 12 (the arrow Z direction). In the stacked state, the second space portion 30B2, the third space portion 30C3, and the fourth space portion 30D4 form a continuous space, and communicate with the outside of the test strip 12 via the first space portion 30A2. The fourth space portion 30D4 is formed in substantially the same shape as the second space portion 30B2 and the third space portion 30C3.

The reagent placement portion 30D3 is provided between the second straight line portion 30D2 and the fourth space portion 30D4. The reagent placement portion 30D3 forms a space in which a reagent piece 36 to be described later can be placed. The reagent piece 36 is held by the adhesive layer on the third plate body 28C when the test strip 12 is manufactured.

When the fourth plate body 28D is stacked on the third plate body 28C, the second straight line portion 30D2 is stacked so as to overlap the first straight line portion 30C2, and forms the flow path 38. At this time, a length of the second straight line portion 30D2 in a direction of the flow path 38 (a length in the arrow X direction) is shorter than the first straight line portion 30C2 by a length of the reagent placement portion 30D3. With such a configuration, if a thickness of a thin plate film forming the reagent piece 36 is appropriately adjusted, the length of the flow path 38 in the arrow Z direction can be changed in the middle of the flow path 38. In particular, when the length of the flow path 38 in the arrow Z direction is reduced in the middle of the flow path 38, the blood can flow efficiently.

The fifth plate body 28E is stacked on the fourth plate body 28D in the arrow Z2 direction. No cutout is provided on the first end side of the fifth plate body 28E, and no partial space 30 is provided at a position facing the second straight line portion 30D2. Accordingly, in the test strip 12, the flow path 38 through which the blood flows is formed in the first straight line portion 30C2 and the second straight line portion 30D2 between the second plate body 28B and the fifth plate body 28E.

The partial space 30 of the fifth plate body 28E includes a reagent insertion portion 30E1 closer to the first end and on the inner side in the width direction, and a fifth space portion 30E2 communicating with the reagent insertion portion 30E1. The reagent insertion portion 30E1 faces the reagent placement portion 30D3 and is formed in the same shape as the reagent placement portion 30D3. The fifth space portion 30E2 faces the fourth space portion 30D4 and is formed in the same shape as the fourth space portion 30D4.

A hydrophilic coating (not illustrated) is applied to surfaces of the second plate body 28B and the fifth plate body 28E. Accordingly, the blood can easily flow in the partial space 30 sandwiched between the second plate body 28B and the fifth plate body 28E.

By stacking the first to fifth plate bodies 28A to 28E described above, the cavity portion 22 of the test strip 12 includes the intake portion 24, the flow path 38 that communicates with the intake portion 24 and through which the blood can flow, and the air vent portion 40 for venting the air from the flow path 38. More specifically, the intake portion 24 is formed by the first cutout portion 30A1, the second cutout portion 30B1, the third cutout portion 30C1, and the fourth cutout portion 30D1. One surface of the intake portion 24 in the arrow Z direction is covered by the fifth plate body 28E. The other surface of the intake portion 24 in the arrow Z direction is open. When an entire surface of the first plate body 28A is the light shielding portion 32, and the second plate body 28B, the third plate body 28C, the fourth plate body 28D, and the fifth plate body 28E are formed of transparent members, because the intake portion 24 is transparent in a plan view of the test strip 12 in the arrow Z direction, the intake portion 24 is easily visible to the patient.

The flow path 38 is formed by the first straight line portion 30C2, the second straight line portion 30D2, and the reagent placement portion 30D3. The air vent portion 40 is constituted by the first to fifth space portions 30A2, 30B2, 30C3, 30D4, and 30E2. The air vent portion 40 discharges the air pushed out from the inside of the flow path 38 when the sample flows through the flow path 38.

The main body portion 20 includes a light shielding layer (the first plate body 28A), spacer layers (the third plate body 28C and the fourth plate body 28D) that form the flow path 38, a first cover layer (the second plate body 28B) and a second cover layer (the fifth plate body 28E) that cover upper and lower surfaces of the spacer layers.

The reagent piece 36 is disposed on the flow path 38 upstream of the air vent portion 40 of the test strip 12 (between the third plate body 28C and the fourth plate body 28D). The reagent piece 36 supports a reagent portion 42 in which a reagent that reacts with the blood in the flow path 38 (colors the blood) is applied on a thin film. Various polymers may be further disposed on the reagent piece 36 depending on properties of the reagent and the measurement system. In the present embodiment, the reagent piece 36 is constituted as a member separated from the plate body 28, whereas the invention is not limited thereto. For example, the reagent piece 36 may be configured by applying the reagent to an appropriate position of a predetermined plate body 28 (for example, the second plate body 28B). Alternatively, the reagent may be applied to a region corresponding to the reagent placement portion 30D3 instead of cutting out the reagent placement portion 30D3 as the space in which the reagent piece 36 is disposed. In this case, the reagent is applied to a region between the second straight line portion 30D2 and the fourth space portion 30D4 in the fourth plate body 28D.

The reagent piece 36 is formed in a rectangular shape extending long in a short direction of the test strip 12 (the width direction, that is, the arrow Y direction) and extending short in the longitudinal direction (the arrow X direction). Specifically, the length of the reagent piece 36 in the arrow Y direction is longer than a flow path width of the flow path 38 upstream of the reagent piece 36. Accordingly, even if the length of the test strip 12 in the arrow Y direction is reduced to 10 mm or less, the reagent piece 36 can be reliably adhered to the third plate body 28C. Further, the measurement opening 34 of the first plate body 28A overlaps the reagent piece 36 according to the present embodiment in a stacking direction, thus the measurement spot 44 (the detection target position) is set to be irradiated with the measurement light of the blood glucose meter 16. The measurement spot 44 will be described in detail later.

Returning to Fig. 1, next, the blood glucose meter 16 to which the test strip 12 is attached will be described. The blood glucose meter 16 is constituted as a reusable type that is capable of repeatedly performing blood glucose measurement. A housing 46 of the blood glucose meter 16 includes a box body portion 50 that has a size that is easy for the user to perform a gripping operation and accommodates a control unit 48 of the blood glucose meter 16 therein, and a cylindrical light measurement unit 52 that protrudes from the box body portion 50 to a front end direction (first end direction) and accommodates the measurement unit 18 of an optical system therein. A power supply button 54, an operation button 56, and a display 58 are provided on an upper surface of the box body portion 50, and an eject lever 60 as an operation portion for removing the test strip 12 after use is provided on an upper surface of the light measurement unit 52.

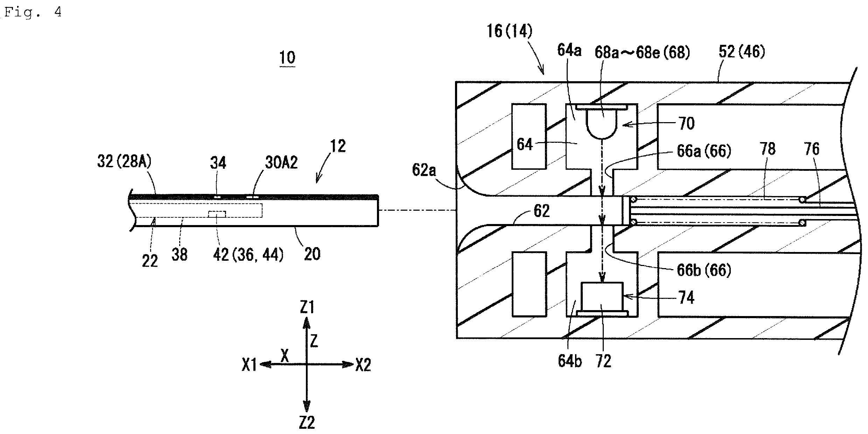

The power supply button 54 switches between activation and deactivation of the blood glucose meter 16 under operation of the user. The operation button 56 functions as an operation portion for performing measurement and display of the blood glucose level based on the operation of the user, switching the display of a measurement result (including a past blood glucose level), and the like in the blood glucose meter 16 in an activation state. Depending on a setting of operation setting software of the blood glucose meter 16, the power supply button 54 may be omitted. The operation button 56 may also function as the power supply button 54. The display 58 is constituted by a liquid crystal, an organic EL, or the like, and displays information to be provided to the user in the measurement of the blood glucose level, such as a display of the blood glucose level or an error display. The eject lever 60 is provided so as to be movable in a front end and base end directions, and is connected to an eject pin 76 (see Fig. 4) provided in the light measurement unit 52.

Meanwhile, the light measurement unit 52 of the blood glucose meter 16 is provided in the box body portion 50. As illustrated in Fig. 4, the light measurement unit 52 is provided with an insertion hole 62 into which the test strip 12 is inserted. The insertion hole 62 is opened in a front end surface (a first end surface) of the blood glucose meter 16, and extends from the first end side of the light measurement unit 52 toward the second end side (a box body portion 50 side). The light measurement unit 52 includes the measurement unit 18 that optically detects glucose in the blood so as to sandwich the insertion hole 62.

The insertion hole 62 corresponds to the test strip 12 and is formed in a rectangular shape in cross section that is long in the width direction and short in an up-down direction of the light measurement unit 52. The insertion hole 62 has an insertion opening 62a in a first end surface of the light measurement unit 52. A dimension of the insertion opening 62a in the width direction and the up-down direction is substantially the same as or slightly larger than a dimension of the test strip 12 in the width direction and the thickness direction.

The light measurement unit 52 has a pair of element accommodation spaces 64 at a position in the middle of extension of the insertion hole 62 inside the light measurement unit 52. The element accommodation space 64 includes a light emitting unit accommodation space 64a and a light receiving unit accommodation space 64b that are provided at positions facing each other with the insertion hole 62 sandwiched therebetween, and an opening 66. The opening 66 is provided between the light emitting unit accommodation space 64a and the light receiving unit accommodation space 64b, and serves as a passage for the measurement light. The measurement unit for the test strip 12 is disposed at a position where the opening 66 and the insertion hole 62 intersect with each other, and the measurement is performed.

The measurement unit 18 includes a light emitting unit 70 in which a plurality of light emitting elements 68 are accommodated in the light emitting unit accommodation space 64a, and a light receiving unit 74 in which a light receiving element 72 is accommodated in the light receiving unit accommodation space 64b. The opening 66 on a light emitting unit 70 side is a circular emitting-side passage opening 66a having a predetermined diameter (dimension). The opening 66 on a light receiving unit 74 side is also a circular light receiving-side passage opening 66b having a predetermined diameter (dimension) (for example, the same dimension as that of the emitting-side passage opening 66a) . The emitting-side passage opening 66a and the light receiving-side passage opening 66b are not limited to the circular shape, and may be formed in various shapes.

The light emitting unit 70 is a light source including the light emitting elements 68 corresponding to a measurement wavelength. In the present embodiment, the light emitting unit 70 includes a plurality of light emitting elements 68 (first to fifth light emitting elements 68a to 68e). The plurality of light emitting elements 68 have a cross shape in a plan view in which three light emitting elements 68 are disposed along a longitudinal direction of the light measurement unit 52, and the other two light emitting elements 68 are disposed adjacent to each other on both sides in a width direction orthogonal to the longitudinal direction with the light emitting element 68 at a center in the longitudinal direction as a base point.

The first to fifth light emitting elements 68a to 68e are configured to emit measurement light having peak wavelengths different from one other in a range of 495 nm to 950 nm. As an example, the first light emitting element 68a emits first measurement light having a peak wavelength of around 650 nm. The second light emitting element 68b emits second measurement light having a peak wavelength of around 900 nm. The third light emitting element 68c emits third measurement light having a peak wavelength of around 800 nm. The fourth light emitting element 68d emits fourth measurement light having a peak wavelength of around 545 nm. The fifth light emitting element 68e emits fifth measurement light having a peak wavelength of around 590 nm. Examples of elements that emit this type of measurement light include LEDs, organic ELs, and laser diodes. The control unit 48 of the blood glucose meter 16 supplies a drive current to cause the first to fifth light emitting elements 68a to 68e to emit light at predetermined timings.

On the other hand, the light receiving unit 74 includes one light receiving element 72. The measurement light emitted from the light emitting elements 68 passes through the emitting-side passage opening 66a, is transmitted through the measurement spot 44 through the measurement opening 34 of the test strip 12, passes through the light receiving-side passage opening 66b, and is received by the light receiving element 72 as detection light (transmitted light). The light receiving element 72 includes, for example, a photodiode (PD).

A second end of the insertion hole 62 is provided with the eject pin 76 connected to the eject lever 60. When the eject pin 76 is pushed in the second end direction as the test strip 12 is inserted by the user, the eject pin 76 is locked (fixed) by a lock mechanism (not illustrated) provided in the housing 46, and a spring 78 contracts. When the eject lever 60 is operated in the first end direction by the user, the lock mechanism that fixes the eject pin 76 is unlocked, and the eject pin 76 can be advanced in the first end direction. By advancing the eject pin 76 in the first end direction while using an elastic restoring force of the spring 78, the test strip 12 attached to the insertion hole 62 can be easily ejected from the insertion hole 62.

Returning to Fig. 1, the control unit 48 of the blood glucose meter 16 is constituted by a control circuit (computer) having a processor, a memory, and an input and output interface (not illustrated). For example, under the operation of the user on the power supply button 54, the control unit 48 drives the measurement unit 18 to calculate the blood glucose level based on a signal corresponding to the amount (or concentration) of glucose in the blood, and displays the calculated blood glucose level on the display 58.

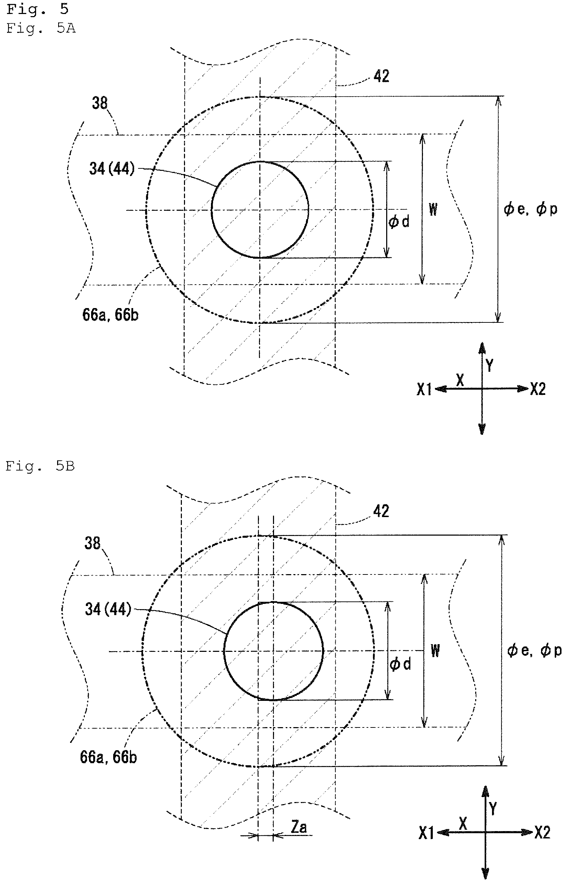

Next, a relationship between the measurement spot 44 in the test strip 12 and the measurement unit 18 of the blood glucose meter 16 will be described with reference to Figs. 3, 5A, and 5B. In the component measurement system 10 according to the present embodiment, the measurement spot 44 is a place where a substance to be measured is present, and is defined by a shape and an area of the measurement opening 34 of the test strip 12. More specifically, in the measurement light emitted from the blood glucose meter 16, a region (area) corresponding to a measurement region in the test strip 12 (that is, the measurement spot 44) is defined by the measurement opening 34. That is, because the test strip 12 uses the black film member as the first plate body 28A, the first plate body 28A functions as the light shielding portion 32 that shields at least a part of the measurement light. In addition, the measurement opening 34 functions as an aperture that defines the measurement spot 44 on the test strip 12. That is, because the aperture and the measurement spot 44 can be adjacent to each other, a distance between the aperture and the measurement spot 44 can be kept short and within a certain range. Therefore, measurement accuracy can be improved. In the present embodiment, the measurement spot 44 is defined in a region overlapping the reagent portion 42.

The material constituting the light shielding portion 32 (the black film member) is not particularly limited as long as the material can appropriately shield the measurement light. For example, the light shielding portion 32 may be constituted by mixing a pigment such as carbon black with a resin such as polyethylene terephthalate (PET), polymethyl methacrylate (PMMA), polycarbonate, polystyrene, polypropylene, acrylonitrile-butadiene-styrene copolymer (ABS), cycloolefin polymer (COP), or cyclic olefin copolymer (COC). The second to fifth plate bodies 28B to 28E may be formed of a resin material having transparency, and for example, the resin material that is not mixed with the pigment can be applied. In the test strip 12, at least one of the second to fifth plate bodies 28B to 28E may be formed of a black film member (the light shielding portion 32).

A light shielding ratio of the light shielding portion 32 is preferably 90% or more based on a measurement method of JIS K7605; 1976 (discontinued standard). In the present embodiment, the black film member having a light shielding rate of 99% or more is applied. In the test strip 12, the entire surface of the first plate body 28A serves as the light shielding portion 32. The test strip 12 is not limited to one having the light shielding portion 32 on the entire surface. In the test strip 12, at least a portion (a peripheral portion of the measurement opening 34) facing the emitting-side passage opening 66a and the light receiving-side passage opening 66b of the blood glucose meter 16 may be the light shielding portion 32. In the present embodiment, in order to detect the transmitted light transmitted through the measurement spot 44 on the reagent piece 36 (reagent portion 42), at least a part of the test strip 12 may be the light shielding portion 32 in the plan view of the test strip 12. That is, in the plan view of the test strip 12, at least a periphery of the measurement spot 44 may be the light shielding portion 32, whereas it is more preferable that the entire surface of the test strip 12 is the light shielding portion 32.

The test strip 12 is not limited to one in which the first plate body 28A is the light shielding portion 32. For example, instead of the first plate body 28A, the fifth plate body 28E on the opposite side of the first plate body 28A may be used as the light shielding portion. Alternatively, the light shielding portion 32 may be provided in any one of the second to fourth plate bodies 28B to 28D stacked in the test strip 12. When any one of the plate bodies 28 constituting the test strip 12 is the light shielding portion 32 having the measurement opening 34, the measurement spot 44 can be defined. The measurement opening 34 may be provided in the plate body 28 configured as the light shielding portion 32. In the present embodiment, by providing the light shielding portion 32 in the first plate body 28A, the test strip 12 can be made to have a thin thickness structure in order to reduce the amount of blood required for measurement while having a configuration in which the flow path 38, the reagent portion 42 applied at the position of the measurement spot 44, and the air vent portion 40 are provided inside the test strip 12.

The measurement opening 34 is formed in a substantially regular circular shape. In this case, a maximum dimension φd of the measurement opening 34 in a direction (that is, a width direction of the flow path 38) orthogonal to a direction from the first end to the second end (a diameter φd in the present embodiment, because an opening shape of the measurement opening 34 is substantially circular in a top plan view) is smaller than that of the first space portion 30A2 having a regular circular shape that is also formed in the first plate body 28A. The maximum dimension φd corresponds to a width dimension of the measurement opening 34. In the width direction of the flow path 38, the maximum dimension φd is smaller than a length in the width direction in which the reagent is applied, and is smaller than a width dimension W of the flow path 38. Instead of cutting out the first plate body 28A, a region of the measurement opening 34 corresponding to the measurement opening 34 may be transparent.

Further, in the width direction of the flow path 38 (the arrow Y direction), the maximum dimension φd of the measurement opening 34 is smaller than a minimum dimension φe of the emitting-side passage opening 66a and a minimum dimension φp of the light receiving-side passage opening 66b. Here, the minimum dimensions φe and φp of the emitting-side passage opening 66a and the light receiving-side passage opening 66b refer to smallest dimensions among line segments passing through the center of each opening 66 and defined by the edge portions of the opening 66. When the opening 66 has a regular circular shape, the minimum dimensions φe and φp are diameters, and when the opening 66 has an elliptical shape, the minimum dimensions φe and φp are dimensions of a minor axis.

For example, in the present embodiment, the maximum dimension φd of the measurement opening 34 is preferably 0.2 mm to 0.8 mm, and most preferably 0.5 mm. On the other hand, the width dimension W of the flow path 38 is preferably 0.3 mm to 2.0 mm, more preferably 0.4 mm to 1.5 mm, and most preferably 1.1 mm ± 0.05 mm. Therefore, the test strip 12 has the reagent portion 42 having the width dimension W of 1.05 mm to 1.15 mm in the flow path 38.

In the blood glucose meter 16, the minimum dimension φe of the emitting-side passage opening 66a and the minimum dimension φp of the light receiving-side passage opening 66b are preferably 0.8 mm to 3.0 mm, more preferably 1.0 mm to 1.5 mm, and most preferably 1.32 mm ± 0.05 mm.

A ratio of the maximum dimension φd of the measurement opening 34 to the width dimension W of the flow path 38 (the length in the arrow Y direction) is preferably 5% to 72%, more preferably 18% to 72%, and still more preferably 27% to 45%. Accordingly, the measurement opening 34 of the test strip 12 reliably faces the reagent portion 42, and the measurement spot 44 of the measurement light can be defined. When the ratio is less than 5%, an error increases due to influence of dust, which is not preferable. When the ratio is greater than 72%, the test strip 12 is easily affected by positional deviation when the test strip 12 is inserted into the blood glucose meter 16. The dimensions of the emitting-side passage opening 66a and the light receiving-side passage opening 66b are larger than the dimension of the measurement opening 34. Because an opening area of the emitting-side passage opening 66a is larger than that of the measurement opening 34, the measurement spot 44 is always defined by the measurement opening 34. Therefore, even if the test strip 12 is attached to the blood glucose meter 16 with the front and back sides thereof reversed, the measurement spot 44 can be defined by the measurement opening 34.

Then, in the component measurement system 10, in a state in which the test strip 12 is attached to the blood glucose meter 16, basically, as illustrated in Fig. 5A, it is preferable that the center of the measurement opening 34 (the measurement spot 44) and the center of the emitting-side passage opening 66a and the light receiving-side passage opening 66b substantially coincide with each other.

Thus, as illustrated in Fig. 5B, in a state where the test strip 12 is attached to the blood glucose meter 16, even if the measurement opening 34 is attached with a largest deviation (deviated by a deviation amount Za), the entire measurement opening 34 can be disposed between the emitting-side passage opening 66a and the light receiving-side passage opening 66b. Accordingly, the measurement spot 44 can receive the measurement light of a consistent light amount from the emitting-side passage opening 66a.

The maximum dimension φd of the measurement opening 34 may be appropriately designed in accordance with the light amount of the measurement light emitted from the light emitting elements 68 or the like. Further, the maximum dimension φd of the measurement opening 34 may be designed in a range of 0.2 mm to 0.8 mm, and in this case, a ratio of the maximum dimension φd of the measurement opening 34 to the minimum dimensions φe and φp of the emitting-side passage opening 66a and the light receiving-side passage opening 66b is set to 15% to 62%. When the ratio is greater than 62%, the measurement opening 34 does not fit inside the emitting-side passage opening 66a and the light receiving-side passage opening 66b. On the other hand, when the ratio is smaller than 15%, for example, when dust is present in the opening 66 corresponding to a passage of the measurement light or the detection light or in the measurement opening 34, the detection light decreases, and thus a correct measurement value cannot be obtained.

When the maximum dimension φd of the measurement opening 34 is 0.2 mm to 0.8 mm, the ratio of the maximum dimension φd to the width dimension W of the flow path 38 (for example, 1.1 mm) is 18% to 72%. When the ratio is greater than 72%, the measurement opening 34 may not fit in the reagent portion 42. On the other hand, when the ratio is smaller than 18%, when the dust is present in the measurement opening 34, the measurement is likely to be affected by the dust.

The test strip 12 and the component measurement system 10 according to the present embodiment are basically configured as described above, and operations thereof will be described below.

The test strip 12 is disposed at a position where the measurement opening 34 overlaps the emitting-side passage opening 66a and the light receiving-side passage opening 66b. At this stage, the test strip 12 is restricted from entering an inner portion of the component measurement device 14 by the eject pin 76.

In this state, the blood glucose meter 16 emits the measurement light having a predetermined wavelength from the light emitting unit 70, receives, by the light receiving unit 74, the transmitted light transmitted through the test strip 12 as the detection light, and calculates the blood glucose level. Here, the component measurement device 14 according to the present embodiment includes the measurement opening 34 on the test strip 12. That is, an irradiation range (the measurement spot 44) of the measurement light with which the reagent portion 42 is irradiated is not defined by the emitting-side passage opening 66a and the light receiving-side passage opening 66b on the blood glucose meter 16 side, and the measurement spot 44 is defined by the measurement opening 34 on the test strip 12.

As described above, because the measurement opening 34 is disposed at the position facing the emitting-side passage opening 66a and the light receiving-side passage opening 66b in the state in which the test strip 12 is attached to the blood glucose meter 16, the measurement light of a consistent light amount can be guided to the measurement spot 44 on the test strip 12. That is, a subject can be irradiated with the light amount defined by the measurement opening 34 at the measurement spot 44. By adopting a configuration in which the measurement opening that defines the measurement spot 44 is not provided on the blood glucose meter 16 side, variation of the measurement position on the test strip 12 can be prevented. In particular, when the transmitted light measurement is performed using the disposable test strip 12 and the blood glucose meter 16, the position of the measurement spot 44 on the test strip 12 is likely to vary due to the deviation for each test strip 12. When the transmitted light measurement is performed using the position at which the reagent is applied (reagent portion 42) as the measurement spot 44, the measurement spot 44 is position-deviated, and thus the measurement spot 44 is affected by a mixing and dissolution state of the blood and the reagent, and the measurement accuracy is reduced. In the disclosure, because alignment between the region to which the reagent is applied (reagent portion 42) and the measurement spot 44 can be precisely controlled in the test strip 12, it is possible to improve the measurement accuracy, particularly reproducibility. With the above configuration, arrangement of a collimator lens in the blood glucose meter 16 can be omitted.

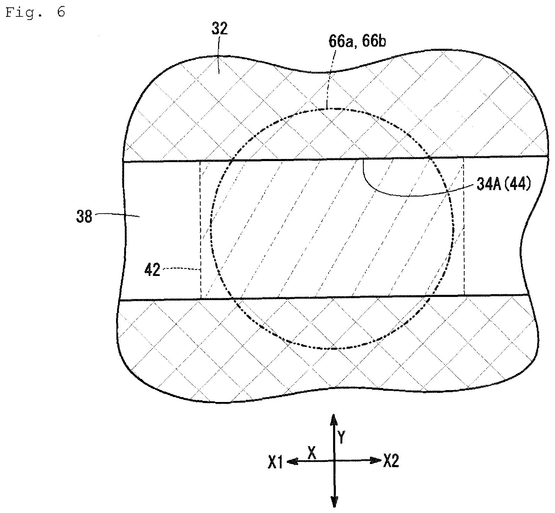

The disclosure is not limited to the above described embodiment, and various modifications can be made within the scope of the invention as defined by the appended claims. For example, as in a first modification illustrated in Fig. 6, the light shielding portion 32 of the test strip 12 may include a measurement opening 34A that defines both sides of the flow path 38 (the reagent portion 42) in the width direction (the arrow Y direction) and does not define the measurement spot 44 in the longitudinal direction orthogonal to the width direction. When at least both sides of the measurement spot 44 in the width direction are defined by the measurement opening 34A, stray light wrapping around from both sides of the measurement spot 44 in the width direction can be reduced, which can contribute to improvement in accuracy. In the test strip 12 having the measurement opening 34A, the light amount of the measurement light can also be stabilized.

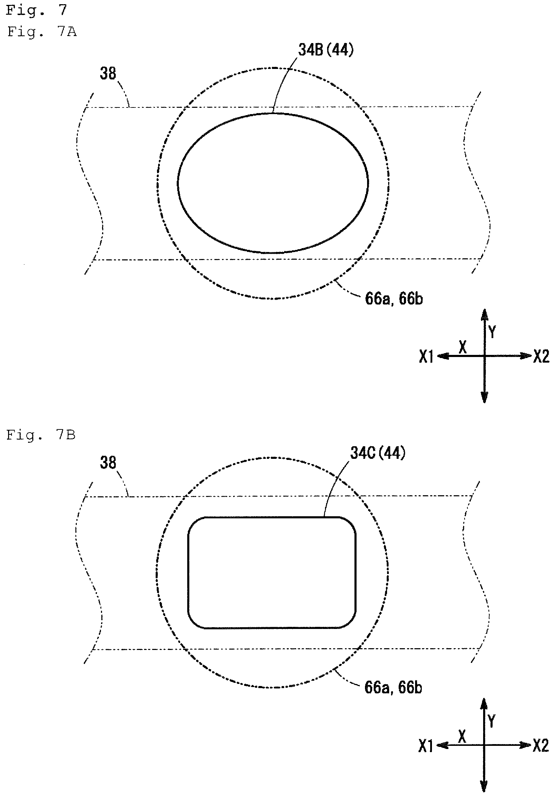

The shape of the measurement opening 34 is not limited to the above-described regular circular shape. For example, as in a second modification illustrated in Fig. 7A, a measurement opening 34B may be formed in an elliptical shape having a major axis in an extending direction of the flow path 38. Alternatively, as in a third modification illustrated in Fig. 7B, a measurement opening 34C may be formed in a rectangular shape having a long side in an extending direction of the flow path 38. In this case, if the measurement opening 34 is configured to fit inside the emitting-side passage opening 66a and the light receiving-side passage opening 66b of the blood glucose meter 16, the measurement light of a consistent light amount can also be always guided to the measurement spot 44. For example, the elliptical measurement opening 34B or the rectangular measurement opening 34C may have a shape in which each of a minor axis (short side) and a major axis (long side) is 70% of that of the emitting-side passage opening 66a and the light receiving-side passage opening 66b, and may have a shape in which each of the minor axis (short side) and the major axis (long side) is 80% of the reagent portion 42.

Further, depending on a type of an object to be measured or a reaction system, the measurement spot 44 formed by the measurement opening 34 of the test strip 12 may not be positioned on the reagent portion 42. For example, the measurement opening 34 may be provided on the second end side (a downstream side) in the arrow Y direction with respect to the reagent portion 42, and a reaction product of the blood and the reagent may be introduced into another measurement site by capillary force to form the measurement spot 44.

Further, the blood glucose meter 16 (the component measurement device 14) is not limited to having the transmissive measurement unit 18 in which the measurement light is emitted from the light emitting unit 70 and the transmitted light transmitted through the test strip 12 is received by the light receiving unit 74. That is, the blood glucose meter 16 may employ a reflective measurement unit 18 in which the light receiving unit 74 detects the measurement light emitted from the light emitting unit 70 and reflected by the test strip 12 (the measurement spot 44). In this case, the test strip 12 may have the measurement opening 34 between the emitting-side passage opening 66a and the flow path 38.

Technical ideas and effects that can be understood from the above described embodiment will be described below.

In the test strip 12, the light shielding portion 32 of the main body portion 20 defines the measurement spot 44 on the reaction product, so that the measurement light of the light amount can be consistently guided to the measurement spot 44. For example, even if the test strip 12 is attached to the component measurement device 14 (the blood glucose meter 16) with deviation in position, the measurement openings 34, 34A to 34C formed in the test strip 12 can also reliably face the light source of the component measurement device 14. That is, a mounting error of the test strip 12 to the component measurement device 14 can be reduced. Accordingly, since the measurement light can be introduced into the region defined by the measurement openings 34, 34A to 34C of the test strip 12, the measurement can be performed at the measurement spot 44 with the most stable accuracy. Accordingly, the variation in the measurement value is reduced.

The light shielding portion 32 is provided on a planar surface portion of the test strip 12, and defines at least both sides of the measurement spot 44 in the width direction. Accordingly, the test strip 12 can prevent the measurement light from being detected by the light receiving unit 74 of the component measurement device 14 (stray light) in a state of wrapping around to the outside in the width direction of the measurement spot 44. Therefore, the test strip 12 can further improve the measurement accuracy.

The light shielding portion 32 has the measurement opening 34 that defines the measurement spot 44, and the width dimension of the measurement opening 34 along the width direction of the main body portion 20 (the maximum dimension φd) is smaller than the width dimension W (the length in the arrow Y direction) of the reagent portion 42 (the flow path 38). Accordingly, the test strip 12 allows a dimensional error of the measurement opening 34 and a manufacturing error of the test strip 12, and can reliably form the measurement spot 44 of the measurement light in a specific region (in the present embodiment, in the region of the reagent portion 42).

The measurement opening 34 is provided at a position overlapping the reagent portion 42 in a plan view. The measurement light of a light amount defined by the measurement opening 34 can be easily guided to the reaction product generated by the reaction between the sample and the reagent.

The light shielding portion 32 is formed of a black film member having a light shielding ratio of 90% or more. Accordingly, the test strip 12 can absorb the measurement light in the light shielding portion 32 around the measurement opening 34. Therefore, reflected light, scattered light, stray light, and the like other than the detection light reaching the light receiving unit 74 can be reduced, and the blood glucose measurement can be performed with higher accuracy.

The sample is blood, and the reagent portion 42 has a reagent that is colored in accordance with the amount of glucose in the blood. The reaction product generated by the reaction between glucose in the blood and the reagent is colored in a detectable manner. Accordingly, the test strip 12 can guide the measurement light to the blood, which reacts with the reagent, through the measurement opening 34, 34A, 34B or 34C, the component measurement device 14 detects the transmitted light, and thereby the blood glucose level can be measured with high accuracy.

The main body portion 20 includes the first plate body 28A (the light shielding layer), the reagent that reacts with the sample (the blood), the third plate body 28C and the fourth plate body 28D (the spacer layer) provided with the flow path 38 that is provided in the test strip 12 as the cavity portion 22 having the intake portion 24 at one end of the test strip 12 and that introduces the sample (blood) to the measurement spot 44, and the second plate body 28B (the first cover layer) and the fifth plate body 28E (the second cover layer) that cover the upper and lower surfaces of the third plate body 28C and the fourth plate body 28D. The first plate body 28A defines the measurement spot 44 on the reaction product of the reagent and the sample. Accordingly, the measurement light of a consistent light amount can be accurately guided to the measurement spot 44.

The flow path 38 is formed by the second plate body 28B and the fifth plate body 28E covering the upper and lower surfaces of the third plate body 28C and the fourth plate body 28D, and one end of the flow path 38 is opened to the outside of the test strip 12 to form the intake portion 24. Accordingly, the flow path 38 can be easily formed. Further, in the stacking direction (the arrow Z direction), the thickness of the flow path 38 can be changed in the flow path 38.

In the main body portion 20, the first to fifth plate bodies 28A to 28E are stacked to form the air vent portion 40 that is opened in at least one surface of the test strip 12 and through which the air from the flow path 38 is vented. Accordingly, the blood can smoothly flow from the intake portion 24 to the end of the flow path 38.

The component measurement system 10 includes the test strip 12 having the main body portion 20 holding the sample (blood), and the component measurement device 14 to which the test strip 12 is attached and that optically measures the component contained in the sample. The component measurement device 14 is configured to emit the measurement light traveling in the thickness direction of the main body portion 20. The main body portion 20 includes the light shielding portion 32 that shields the measurement light. The light shielding portion 32 includes the measurement opening 34, 34A, 34B or 34C through which the measurement spot 44 of the measurement light is formed on the reaction product of the reagent and the sample. Accordingly, the component measurement system 10 can guide the measurement light of a consistent light amount to the measurement spot 44 with a simple configuration while preventing the stray light, and can perform the component measurement with higher accuracy.

The measurement opening 34 is disposed on an inner side of the emitting-side passage opening 66a through which the measurement light emitted from the component measurement device 14 passes, in a state in which the test strip 12 is attached to the component measurement device 14 (the blood glucose meter 16). That is, the measurement opening 34 is disposed closer to the light receiving unit 74 than the emitting-side passage opening 66a. Accordingly, the measurement opening 34 narrows down the measurement light passing through the emitting-side passage opening 66a without being cutout in the measurement opening 34, and the measurement spot 44 can be formed satisfactorily.

In a state in which the test strip 12 is attached to the component measurement device 14 (the blood glucose meter 16), the measurement opening 34 is disposed inside the light receiving-side passage opening 66b of the light receiving unit 74 of the component measurement device 14 that receives the measurement light transmitted through the main body portion 20. That is, the measurement opening 34 is disposed closer to the light emitting unit 70 than the light receiving-side passage opening 66b. Accordingly, the test strip 12 can more reliably guide the transmitted light transmitted through the measurement spot 44 to the light receiving unit 74.

The component measurement system 10 and the test strip 12 according to the disclosure are not limited to being applied to the blood glucose level measurement system that measures the blood glucose level, and can be applied to various systems that optically measure an analyte component. For example, examples of the analyte to be measured in a medical site include a solution of a sample obtained from a living body such as urine (ketone body or the like), interstitial fluid, or saliva in addition to the blood, and the analyte may be a stock solution or an experimental product to be subjected to a chemical treatment or the like. Alternatively, the component measurement system 10 can also be applied to a device that performs the component measurement of the analyte in such as wastewater or an industrial sample. The same applies to a test strip 12a to be described below.

Next, the test strip 12a according to a modification will be described. In the description of the test strip 12a, the same components as those of the test strip 12 described above are denoted by the same reference numerals, and the description thereof will be omitted.

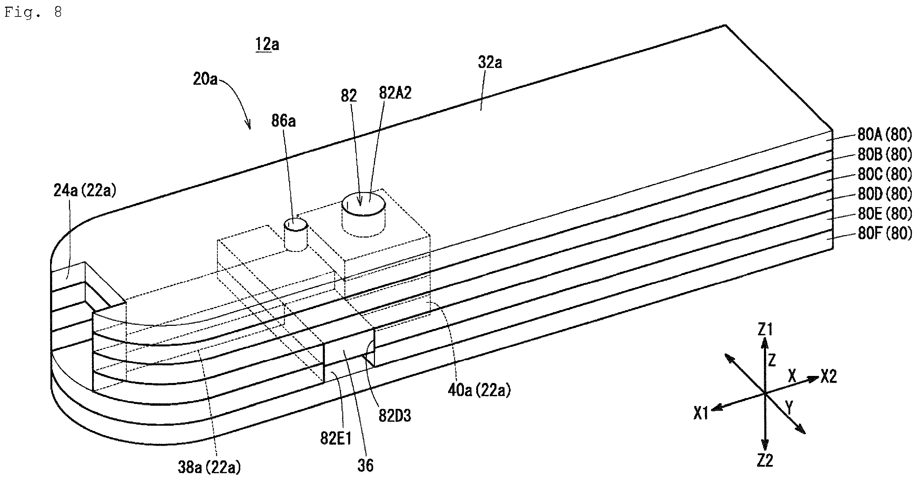

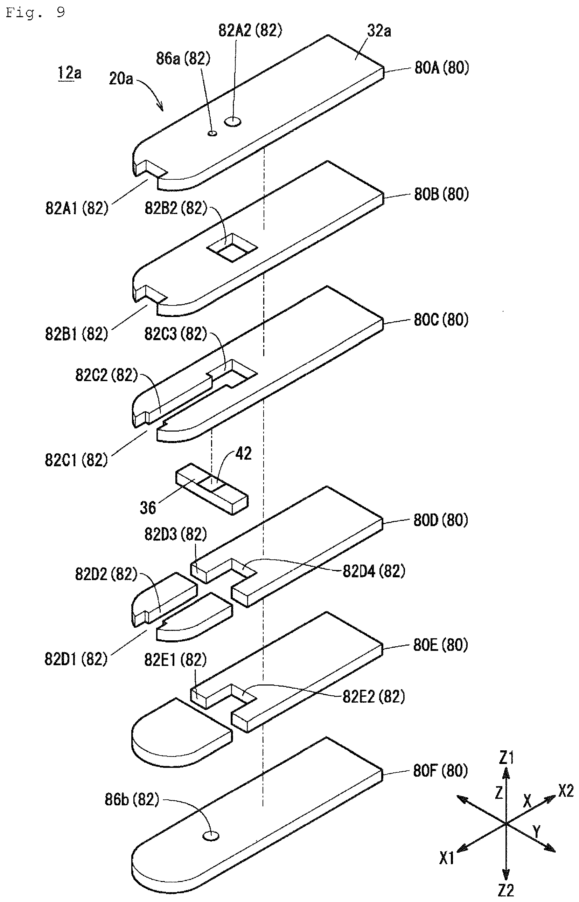

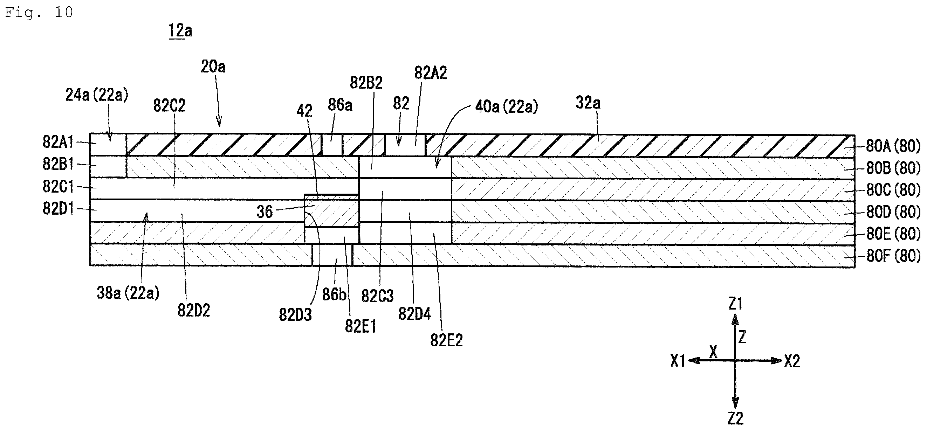

As illustrated in Figs. 8 to 11, a main body portion 20a of the test strip 12a extends in one direction (an arrow X direction). The main body portion 20a is formed by stacking and integrating a plurality of plate bodies 80 in a thickness direction of the plate bodies 80 (an arrow Z direction). Hereinafter, the plurality of plate bodies 80 are referred to as a first plate body 80A, a second plate body 80B, a third plate body 80C, a fourth plate body 80D, a fifth plate body 80E, and a sixth plate body 80F from an upper direction (an arrow Z1 direction) toward a lower direction (an arrow Z2 direction) in Fig. 9. Outer edges of the first to sixth plate bodies 80A to 80F are formed in substantially the same shape (a substantially rectangular shape having an arc on the first end side) in a plan view from the arrow Z direction.

Each of the first to sixth plate bodies 80A to 80F is cut out before being stacked, and therefore the first to sixth plate bodies 80A to 80F each has a partial space 82 that extends in the thickness direction and is formed in an appropriate shape. The partial spaces 82 form a cavity portion 22a, a first measurement opening 86a (aperture), a second measurement opening 86b, and a first space portion 82A2 of the test strip 12a in a state in which the first to sixth plate bodies 80A to 80F are stacked. An adhesive layer (not illustrated) made of an adhesive or the like is provided between the plate bodies 80 adjacent to each other. The adjacent plate bodies 80 are firmly adhered to each other by the adhesive layer.

The first plate body 80A is a planar member disposed at an end on one side in the thickness direction of the test strip 12a (an end in the arrow Z1 direction). A surface of the first plate body 80A in the arrow Z2 direction has an adhesive layer. The first plate body 80A is configured as a black film member, and constitutes a light shielding portion 32a that shields the measurement light in the test strip 12a. The partial space 82 of the first plate body 80A includes a first cutout portion 82A1 provided on the first end side (the end in the arrow X1 direction), the first measurement opening 86a, and the first space portion 82A2.

The first cutout portion 82A1 forms a part of an intake portion 24a. The first cutout portion 82A1 is formed in a quadrangular shape in a plan view from the arrow Z direction. The first measurement opening 86a and the first space portion 82A2 are formed in the same manner as the measurement opening 34 and the first space portion 30A2 described above. Therefore, description of the configurations of the first measurement opening 86a and the first space portion 82A2 will be omitted.

The second plate body 80B is stacked on the first plate body 80A on the other side (the arrow Z2 direction) in the thickness direction of the test strip 12a. The second plate body 80B is a film-shaped member, and at least a part of the second plate body 80B is transparent. The partial space 82 of the second plate body 80B includes a second cutout portion 82B1 on the first end side, and a second space portion 82B2 closer to the first end side of the second plate body 80B and on an inner side in the width direction (the arrow Y direction).

The second cutout portion 82B1 forms a part of the intake portion 24a. The second cutout portion 82B1 is formed in a quadrangular shape (the rectangular shape in the illustrated example) in the plan view from the arrow Z direction. The second cutout portion 82B1 communicates with the first cutout portion 82A1 in the arrow Z2 direction. The second cutout portion 82B1 is formed in the same size and shape as the first cutout portion 82A1. The second space portion 82B2 is formed in the same manner as the second space portion 30B2 described above. Therefore, description of the configuration of the second space portion 82B2 will be omitted.

In the second plate body 80B, the partial space 82 is not provided at a portion facing the first measurement opening 86a of the first plate body 80A. A portion of the second plate body 80B facing the first measurement opening 86a is formed of a transparent member. Accordingly, the second plate body 80B forms a liquid-tight barrier between the first measurement opening 86a and the partial space 82 of the third plate body 80C.

The third plate body 80C is a film-shaped member stacked on the second plate body 80B in the arrow Z2 direction. Both surfaces of the third plate body 80C in the thickness direction (the arrow Z direction) have adhesive layers. The partial space 82 of the third plate body 80C includes a third cutout portion 82Cl, a first straight line portion 82C2, and a third space portion 82C3. The third cutout portion 82C1, the first straight line portion 82C2, and the third space portion 82C3 are formed in the third plate body 80C as spaces that are continuous from the first end toward the second end side.

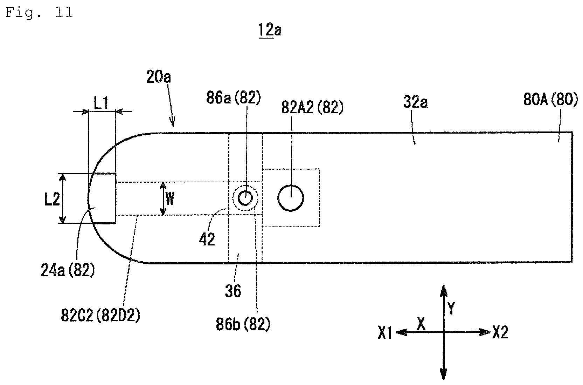

The third cutout portion 82C1 forms a part of the intake portion 24a. The third cutout portion 82C1 is formed in a quadrangular shape (the rectangular shape in the illustrated example) in the plan view as viewed from the arrow Z direction. The third cutout portion 82C1 communicates with the second cutout portion 82B1 in the arrow Z2 direction. The third cutout portion 82C1 has the same area and the same shape as the first cutout portion 82Al and the second cutout portion 82B1. A length L2 of the third cutout portion 82C1 along the width direction of the third plate body 80C (the arrow Y direction) is longer than a width dimension W of the first straight line portion 82C2 (a length in the arrow Y direction) along the width direction of the third plate body 80C (see Fig. 11). The first straight line portion 82C2 and the third space portion 82C3 are formed in the same manner as the first straight line portion 30C2 and the third space portion 30C3 described above. Therefore, description of the configurations of the first straight line portion 82C2 and the third space portion 82C3 will be omitted.

The fourth plate body 80D is stacked on the third plate body 80C in the arrow Z2 direction. A surface of the fourth plate body 80D in the arrow Z2 direction has an adhesive layer. The partial space 82 of the fourth plate body 80D includes a fourth cutout portion 82D1, a second straight line portion 82D2, a reagent placement portion 82D3, and a fourth space portion 82D4.

The fourth cutout portion 82D1, the second straight line portion 82D2, the reagent placement portion 82D3, and the fourth space portion 82D4 are connected from the first end toward the second end side in the arrow X direction to form a continuous space. The fourth cutout portion 82D1 is formed at a first end of the partial space 82, and forms a part of the intake portion 24a. The fourth cutout portion 82D1 communicates with the third cutout portion 82C1 in the arrow Z2 direction. The second straight line portion 82D2 and the fourth space portion 82D4 are formed in the same manner as the second straight line portion 30D2 and the fourth space portion 30D4 described above. Therefore, description of the configurations of the second straight line portion 82D2 and the fourth space portion 82D4 will be omitted.

The reagent placement portion 82D3 is provided between the second straight line portion 82D2 and the fourth space portion 82D4. The reagent placement portion 82D3 extends in a rectangular shape over an entire width of the fourth plate body 80D (an entire length in the arrow Y direction) . Therefore, the fourth plate body 80D is divided into three parts by forming the fourth cutout portion 82D1, the second straight line portion 82D2, and the reagent placement portion 82D3.

The fifth plate body 80E is stacked on the fourth plate body 80D in the arrow Z2 direction. A first end side of the fifth plate body 80E is not provided with a cutout, and is formed in a semicircular shape in the plan view from the arrow Z direction. The fifth plate body 80E is neither provided with the partial space 82 at a position facing the second straight line portion 82D2. Accordingly, in the test strip 12a, a flow path 38a through which the blood flows is formed in the first straight line portion 82C2 and the second straight line portion 82D2 between the second plate body 80B and the fifth plate body 80E.

The partial space 82 of the fifth plate body 80E includes a reagent insertion portion 82E1 closer to the first end and a fifth space portion 82E2 communicating with the reagent insertion portion 82E1. The reagent insertion portion 82E1 faces the reagent placement portion 82D3 and is formed to have the same shape. The reagent insertion portion 82E1 communicates with the reagent placement portion 82D3 in the arrow Z2 direction. That is, the reagent insertion portion 82E1 extends over an entire width of the fifth plate body 80E (an entire length in the arrow Y direction). Therefore, the fifth plate body 80E is divided into two parts in the arrow X direction by the reagent insertion portion 82E1.

When the test strip 12a is manufactured, the reagent piece 36 is inserted from the reagent insertion portion 82E1 into the reagent placement portion 82D3 and held on a surface of the third plate body 80C in the arrow Z2 direction. In this case, a width of the test strip 12a in the arrow Y direction can be narrowed without narrowing the widths of the reagent placement portion 82D3 and the reagent insertion portion 82E1 in the arrow Y direction. That is, the test strip 12a can be made compact without reducing an adhesion area of the reagent piece 36 to the third plate body 80C. Because the configuration of the reagent piece 36 is the same as that of Figs. 2 to 7B, description thereof will be omitted.

The sixth plate body 80F is stacked on the fifth plate body 80E in the arrow Z2 direction. The sixth plate body 80F is a planar member disposed at an end in the thickness direction of the test strip 12a (an end in the arrow Z2 direction). A surface of the sixth plate body 80F in the arrow Z1 direction has an adhesive layer. The partial space 82 of the sixth plate body 80F includes the second measurement opening 86b. The sixth plate body 80F forms a liquid-tight cover on the reagent insertion portion 82E1 and the fifth space portion 82E2 from the arrow Z2 direction.

The second measurement opening 86b transmits the measurement light in the thickness direction of the test strip 12a. That is, the second measurement opening 86b penetrates the sixth plate body 80F in the thickness direction. The second measurement opening 86b is positioned in the arrow Z2 direction of the first measurement opening 86a. The second measurement opening 86b is formed in a circular shape. A diameter of the second measurement opening 86b is larger than a diameter of the first measurement opening 86a (see Fig. 11). In other words, the second measurement opening 86b is provided such that the entire first measurement opening 86a is positioned inside the second measurement opening 86b when viewed from the arrow Z direction. The sixth plate body 80F may be manufactured of a transparent member only in a range corresponding to the second measurement opening 86b.

A hydrophilic coating (not illustrated) is applied to a surface of the second plate body 80B in the arrow Z2 direction and a surface of the fifth plate body 80E in the arrow Z1 direction. Accordingly, the blood can easily flow through the partial space 82 (the flow path 38a) sandwiched between the second plate body 80B and the fifth plate body 80E.

By stacking the first to sixth plate bodies 80A to 80F described above, the cavity portion 22a of the test strip 12a includes the intake portion 24a, the flow path 38a that communicates with the intake portion 24a and through which the blood can flow, and the air vent portion 40a for venting the air from the flow path 38a. More specifically, the intake portion 24a is formed by the first cutout portion 82A1, the second cutout portion 82B1, the third cutout portion 82Cl, and the fourth cutout portion 82D1. The flow path 38a is formed by the first straight line portion 82C2, the second straight line portion 82D2, and the reagent placement portion 82D3. The air vent portion 40a is constituted by the first to fifth space portions 82A2, 82B2, 82C3, 82D4, and 82E2. The air vent portion 40a is designed to be connected to the flow path 38a and to discharge the air pushed out by the blood from the flow path 38a. The sixth plate body 80F is further stacked on the first to fifth plate bodies 80A to 80E to seal one surface of the air vent portion 40a. Accordingly, risk of leakage of the blood into the component measurement device 14 can be reduced.

As illustrated in Fig. 11, the intake portion 24a is formed in a quadrangular shape in the plan view as viewed from the arrow Z direction. A ratio of a length L2 of the intake portion 24a along a minor axis direction of the main body portion 20a (a width of the intake portion 24a along the arrow X direction, mm) to a length L1 of the intake portion 24a along a major axis direction of the main body portion 20a (a length in arrow Y direction, mm) is preferably 0.5 or more and 3 or less. The ratio of the length L2 of the intake portion 24a to the width dimension W of the flow path 38a is preferably 0.45 or more and 1.82 or less. In this case, the sample (blood) taken into the intake portion 24a can be guided toward the reagent portion 42 at a high speed. That is, the sample (blood) can consistently flow to the reagent portion 42. Therefore, the reagent can be substantially uniformly dissolved by the sample (blood) (dissolving deviation of the reagent in the reagent portion 42 can be prevented). By increasing an intake speed, a time for the blood to flow in the flow path 38a can be shortened.

The test strip 12a described above is manufactured by cutting out the partial spaces 82 of the first plate body 80A, the second plate body 80B, the third plate body 80C, the fourth plate body 80D, the fifth plate body 80E, and the sixth plate body 80F, and stacking the plate bodies using a double-sided tape or an adhesive. After the first to fifth plate bodies 80A to 80E are bonded, the reagent piece 36 is disposed in a reagent portion arrangement space. Thereafter, the test strip 12a is manufactured by stacking the sixth plate body 80F on the fifth plate body 80E.

A method of manufacturing the test strip 12 described above is similar with a method of manufacturing the test strip 12a. That is, during manufacturing of the test strip 12, the sixth plate body 80F is not stacked after a reagent disposing step.

The test strip 12a has the same effects as those of the test strip 12 described above.

In the test strip 12a, the intake portion 24a is formed in a quadrangular shape in the plan view, and the ratio of the length L2 of the intake portion 24a along the minor axis direction of the main body portion 20a to the length L1 of the intake portion 24a along the major axis direction of the main body portion 20a is 0.5 or more and 3 or less. According to such a configuration, a flow speed of the blood in the test strip 12a can be increased, and spreadability of the blood in the test strip 12a can be improved. The term "spreadability" refers to a degree to which the blood spreads uniformly and quickly in the flow path 38a. In the present embodiment, the blood flowing on the reagent portion 42 in a flow direction of the blood such that a flow end surface of the blood is substantially uniform in the width direction is referred to as the "spreadability". That is, even when viscosity of the blood is relatively high, the blood can consistently flow to the reagent portion 42, and the blood can be substantially uniformly dissolved in the reagent portion 42. By mixing the blood and the reagent portion 42 substantially uniformly, coloration corresponding to the amount of glucose contained in the blood can be accurately implemented in the test strip 12a. When the light shielding portion 32a is applied to the test strip 12a, because it is difficult to visually check the flow of blood, the user may be anxious about success or failure of the measurement. By increasing the flow speed of the blood, the measurement can be quickly performed. Therefore, the user can be promptly notified of the success or failure of the measurement via the component measurement device 14.

In the test strip 12a, the ratio L2 of the length of the intake portion 24a along the minor axis direction of the main body portion 20a to the width dimension W of the flow path 38a is 0.45 or more and 1.82 or less. According to such a configuration, a blood flow rate in the test strip 12a can be made relatively high, and the spreadability of the reagent portion 42 can be improved.

The main body portion 20a includes the sixth plate body 80F that covers at least a portion of the air vent portion 40a. According to such a configuration, even when the blood guided from the intake portion 24a to the flow path 38a flows out to the air vent portion 40a, the sixth plate body 80F can prevent leakage of the blood to the outside of the test strip 12a.

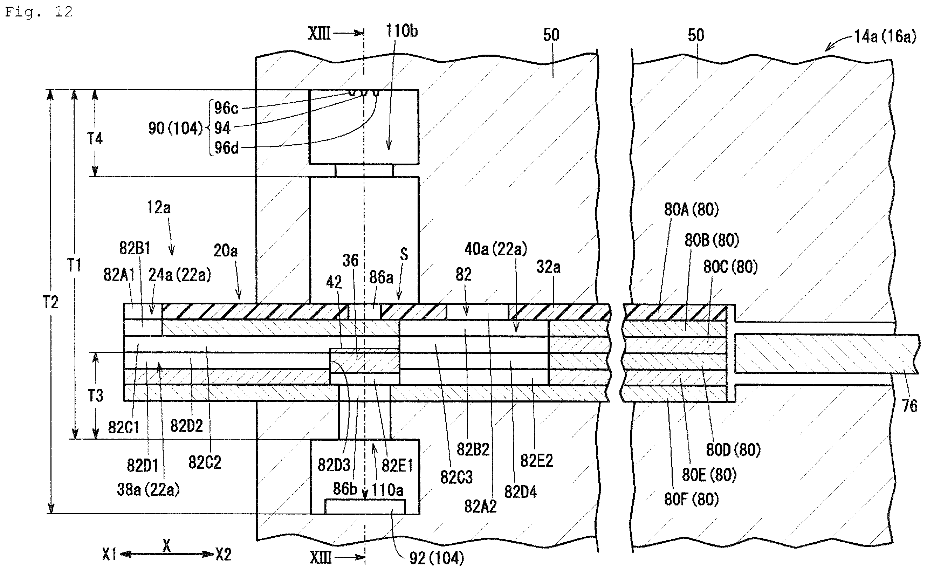

Next, a component measurement device 14a according to a modification will be described. As illustrated in Figs. 12 to 14, the component measurement device 14a is a blood glucose meter 16a to which the test strip 12a and the test strip 12 are attached.

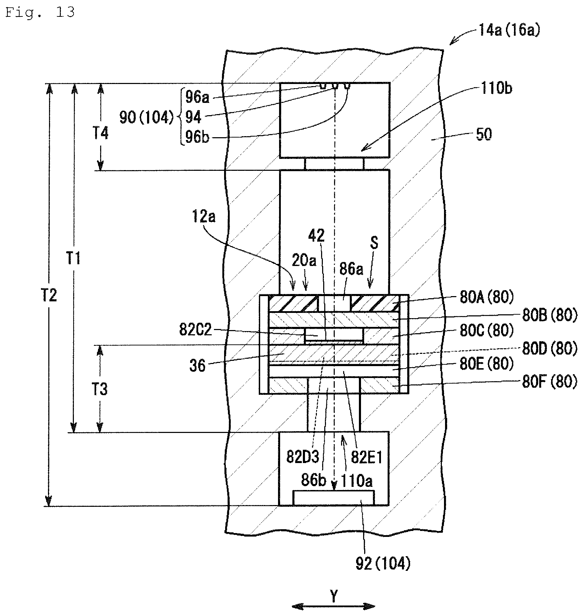

As illustrated in Figs. 12 and 13, the component measurement device 14a includes a light emitting unit 90 and a light receiving unit 92. The light emitting unit 90 and the light receiving unit 92 are disposed to face each other with a chip attachment space S sandwiched therebetween. As illustrated in Figs. 12 and 13, in a state in which the test strip 12a is attached in the chip attachment space S of the component measurement device 14a, the test strip 12a is irradiated with irradiation light emitted by the light emitting unit 90. The light receiving unit 92 receives transmitted light transmitted through the test strip 12a among the irradiation light emitted from the light emitting unit 90 to the test strip 12a.

As illustrated in Figs. 12 to 14, the light emitting unit 90 includes five light sources. Specifically, the light emitting unit 90 includes a first light source 94, a second light source 96a, a third light source 96b, a fourth light source 96c, and a fifth light source 96d. Here, as illustrated in Fig. 13, the first light source 94, the second light source 96a, and the third light source 96b are disposed at different positions in a flow path width direction (the arrow Y direction, both right and left directions in Fig. 13) orthogonal to a flow direction (the arrow X2 direction, the direction toward the right in Fig. 12) in which the blood flows in the flow path 38a of the test strip 12a.

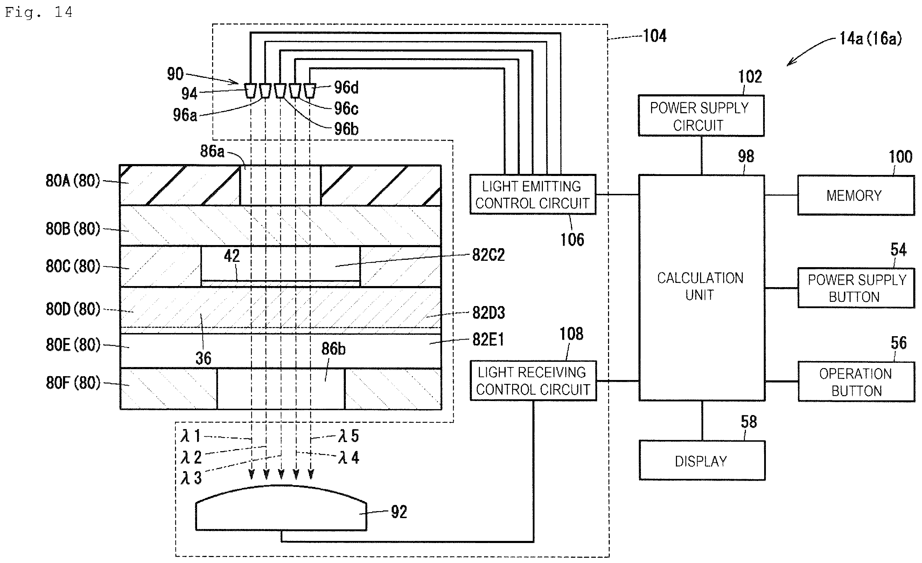

As illustrated in Fig. 14, the component measurement device 14a further includes, in addition to the box body portion 50 (housing), the display 58, the eject pin 76, the power supply button 54, and the operation button 56 that are described above, a calculation unit 98, a memory 100, a power supply circuit 102, and a measurement optical system 104.

The calculation unit 98 is implemented by a microprocessing unit (MPU) or a central processing unit (CPU), and can implement a control operation of each unit by reading and executing a program stored in the memory 100 or the like. The memory 100 is implemented by a non-transient storage medium that is volatile or non-volatile, and can read or write various kinds of data (including a program) necessary for executing the component measurement method illustrated here. In response to the operation of the power supply button 54, the power supply circuit 102 supplies power to or stops the supply of power to each unit in the component measurement device 14a including the calculation unit 98.

The measurement optical system 104 is an optical system capable of acquiring optical characteristics of the reagent portion 42 containing a colored component generated by a color reaction between glucose in the blood and a coloring reagent contained in the reagent. Specifically, the measurement optical system 104 includes the light emitting unit 90, a light emitting control circuit 106, the light receiving unit 92, and a light receiving control circuit 108.

The light emitting unit 90 includes a plurality of light sources. Specifically, the light emitting unit 90 according to the present embodiment includes five light sources that emit irradiation light (for example, visible light or infrared light) having different spectral radiation characteristics. More specifically, the light emitting unit 90 according to the present embodiment includes the first light source 94, the second light source 96a, the third light source 96b, the fourth light source 96c, and the fifth light source 96d. In Fig. 14, for convenience of description, a positional relationship in which five light sources shown as the five light sources including the first to fifth light sources 94 and 96a to 96d are arranged in a line is illustrated in order to illustrate the five light sources, whereas an actual positional relationship among the first to fifth light sources 94 and 96a to 96d is different. The actual positional relationship among the first to fifth light sources 94 and 96a to 96d is a positional relationship illustrated in Figs. 12 and 13.

Peak wavelengths of light emitted from the first to fifth light sources 94 and 96a to 96d are λ1 to λ5, respectively. As the first to fifth light sources 94 and 96a to 96d, various light emitting elements such as light emitting diode (LED) elements, organic electro-luminescence (EL) elements, inorganic EL elements, and laser diode (LD) elements can be applied. As the first to fifth light sources 94 and 96a to 96d, the above-described LED elements are likely to be used in consideration of versatility and the like. Hereinafter, the above-described "peak wavelength" will be described as the wavelength of light emitted from each light source, and for convenience of description, the peak wavelength λ1 of the first light source 94 is described as a "first predetermined wavelength λ1", the peak wavelength λ2 of the second light source 96a is described as a "second predetermined wavelength λ2", the peak wavelength λ3 of the third light source 96b is described as a "third predetermined wavelength A3", the peak wavelength λ4 of the fourth light source 96c is described as a "fourth predetermined wavelength λ4", and the peak wavelength λ5 of the fifth light source 96d is described as a "fifth predetermined wavelength A5".

As illustrated in Fig. 14, the light receiving unit 92 according to the present embodiment is constituted by one light receiving element disposed to face the light emitting unit 90 with the test strip 12a sandwiched therebetween. The light receiving unit 92 receives the transmitted light that is emitted from the first to fifth light sources 94 and 96a to 96d of the light emitting unit 90 to the reagent portion 42 (the measurement spot 44) of the reagent piece 36 of the test strip 12a and transmitted through the test strip 12a. As the light receiving unit 92, various photoelectric conversion elements including photo diode (PD) elements, photoconductors, and phototransistors can be applied.

The light emitting control circuit 106 supplies a drive power signal to each of the first to fifth light sources 94 and 96a to 96d to turn on or off the first to fifth light sources 94 and 96a to 96d. The light receiving control circuit 108 acquires a digital signal (hereinafter referred to as a detection signal) by performing logarithmic conversion and A/D conversion on an analog signal output from the light receiving unit 92.

The component measurement device 14a can measure a component to be measured in the blood based on the optical characteristics of the reagent portion 42 containing a colored component generated by a coloring reaction between the component to be measured in the blood and the reagent. Specifically, the component measurement device 14a can estimate, using irradiation light having the second predetermined wavelength λ2 to the fifth predetermined wavelength λ5, an amount of noise other than the colored component included in a first actual measurement value of absorbance of the reagent portion 42 measured by irradiating the reagent portion 42 with irradiation light having the first predetermined wavelength λ1 as a measurement wavelength. More specifically, the component measurement device 14a can estimate the above-described amount of noise by using a second actual measurement value to a fifth actual measurement value of the absorbance of the reagent portion 42 measured by irradiating the reagent portion 42 with the irradiation light having the second predetermined wavelength λ2 to the fifth predetermined wavelength λ5, and measure the absorbance of the colored component and further the component to be measured.

As illustrated in Figs. 12 and 13, the first to fifth light sources 94 and 96a to 96d are disposed to face the reagent portion 42 positioned in the flow path 38a of the blood. More specifically, the first to fifth light sources 94, 96a to 96d according to the present embodiment are disposed to face a holding position of the reagent portion 42 of the flow path 38a of the blood in a direction (the same direction as the thickness direction of the test strip 12a in the present embodiment) orthogonal to both the flow direction and the flow path width direction.

As illustrated in Fig. 13, the first light source 94 and the second light source 96a are disposed adjacently along the flow path width direction orthogonal to the flow direction of the blood at the position of the reagent portion 42 in the flow path 38a of the blood. With such a configuration, it is easy to set a first irradiation position of the irradiation light from the first light source 94 on the reagent portion 42 and a second irradiation position of the irradiation light from the second light source 96a on the reagent portion 42 to positions where at least a part thereof overlaps in the flow path width direction.

In the present embodiment, as illustrated in Fig. 13, the first light source 94, the second light source 96a, and the third light source 96b are disposed adjacently along the flow path width direction with the first light source 94 as a center. With such an arrangement, not only regions in the flow direction of the two irradiation positions including the first irradiation position of the first light source 94 and the second irradiation position of the second light source 96a, but also regions in the flow direction of two irradiation positions including the first irradiation position and a third irradiation position of the third light source 96b can be easily set to positions where at least a part thereof overlaps in the flow path width direction.

As illustrated in Figs. 12 and 13, the light receiving unit 92 faces the first to fifth light sources 94 and 96a to 96d in the thickness direction with the reagent portion 42 positioned in the flow path 38a of the attached test strip 12a sandwiched therebetween, and as described above, receives the transmitted light in which the irradiation light from the first to fifth light sources 94 and 96a to 96d is transmitted through the reagent portion 42. As illustrated in Figs. 12 and 13, the component measurement device 14a includes a first aperture portion 110a (the light receiving-side passage opening) that is positioned between the reagent portion 42 and the light receiving unit 92 and adjusts the light amount reaching the light receiving unit 92 in the transmitted light transmitted through the reagent portion 42. A difference between an incident angle of the irradiation light from the first light source 94 to the reagent portion 42 and an incident angle of the irradiation light from each of the second to fifth light sources 96a to 96d to the reagent portion 42 affects estimation accuracy of the amount of noise. Therefore, it is preferable to reduce the difference between the incident angle of the irradiation light from the first light source 94 to the reagent portion 42 and the incident angle of the irradiation light from each of the second to fifth light sources 96a to 96d to the reagent portion 42. That is, it is preferable to increase a distance T1 between the first to fifth light sources 94 and 96a to 96d and the first aperture portion 110a in a facing direction (the same direction as the thickness direction of the test strip 12a in Figs. 12 and 13) in order to improve the estimation accuracy of the amount of noise. On the other hand, by reducing a distance T2 between the first to fifth light sources 94 and 96a to 96d and the light receiving unit 92 in the facing direction, it is possible to improve light efficiency and to reduce the size of the component measurement device 14a.

When a deviation (hereinafter, referred to as "measurement visual field difference") between the region of the first irradiation position of the first light source 94 and each of the regions of the second irradiation position to the fifth irradiation position of the second to fifth light sources 96a to 96d is large, measurement portions do not coincide with each other, and thus the accuracy of the measurement result of the component to be measured may decrease. Therefore, it is preferable to reduce the measurement visual field difference. Therefore, it is preferable to shorten a distance T3 between the reagent portion 42 and the first aperture portion 110a in the facing direction (the same direction as the thickness direction of the test strip 12a in Figs. 12 and 13).

Further, as illustrated in Figs. 12 and 13, the component measurement device 14a includes a second aperture portion 110b (the emitting-side passage opening) that is positioned between the first to fifth light sources 94 and 96a to 96d and the reagent portion 42 and adjusts the light amount reaching the reagent portion 42 from the first to fifth light sources 94 and 96a to 96d. In particular, the second aperture portion 110b is preferably designed such that light reflected by an inner wall of the second aperture portion 110b (hereinafter, referred to as "stray light") among the light emitted from the first to fifth light sources 94 and 96a to 96d does not enter the first aperture portion 110a. The light emitted from the first to fifth light sources 94 and 96a to 96d can be considered as being attenuated to 5% by wall surface reflection once and disappear by multi-reflection of three or more times. Therefore, in the present embodiment, if the stray light reflected by the inner wall of the second aperture portion 110b does not reach the first aperture portion 110a and is reflected by some wall surface, the stray light does not enter the first aperture portion 110a due to the multi-reflection. In the present embodiment, an optical axis of each light source is designed to be specularly reflected by the inner wall of the second aperture portion 110b, but is actually diffusely reflected by the inner wall of the second aperture portion 110b, and the stray light has a predetermined distribution. Therefore, in the present embodiment, it is preferable to set a distance T4 (a distance between the first to fifth light sources 94, 96a to 96d and the second aperture portion 110b) or the like such that a difference between the incident angle of the stray light and the incident angle of the first light source 94 is equal to or smaller than a predetermined value even when a part of the stray light enters the first aperture portion 110a.

In this way, by adjusting the positions and the like of the first aperture portion 110a and the second aperture portion 110b, the difference in the incident angle of the irradiation light and the measurement visual field difference are set within a predetermined range. The optical system of the component measurement device 14a does not use a lens such as a condenser lens. When a lens is used, light condensing efficiency can be improved by bringing the lens close to the light source, but it is necessary to maintain a positional relationship between the light source and the lens with high accuracy, high assembly accuracy is required, or an additional step of adjusting a variation in the positional relationship between the light source and the lens is required. Therefore, in the component measurement device 14a, by setting the positions and the like of the first aperture portion 110a and the second aperture portion 110b instead of using the lens, a configuration in which the measurement accuracy is improved without requiring the high assembly accuracy is implemented. The configurations of the first aperture portion 110a and the second aperture portion 110b are the same as those of the light receiving-side passage opening 66b and the emitting-side passage opening 66a in the component measurement system 10 described above, and an opening area of the first aperture portion 110a is smaller than an opening area of the second aperture portion 110b.

Next, tests performed to check the effects of the invention will be described.

### [Sample]

Seven test strips 12a (Examples 1 to 7) according to the disclosure were prepared, and five test strips 12a (Comparative Examples 1 to 5) according to comparative examples were prepared. In Examples 1 to 7 and Comparative Examples 1 to 5, values of the length L1 of the intake portion 24a along the major axis direction of the test strip 12a (the length in the arrow X direction) and the length L2 of the intake portion 24a along the minor axis direction of the test strip 12a (the length in the arrow Y direction) were changed, and other configurations were the same as one another (see Fig. 11). The thickness of the flow path 38a (the length in the arrow Z direction) was set to 90 µm.

### [Test Method]

For Examples 1 to 7 and Comparative Examples 1 to 5, the intake portion 24a was held in a state in which the intake portion 24a is oriented in a direction in which gravity acts (a state in which the gravity acts most), blood was spotted on the intake portion 24a, a flow rate V of the blood flowing through the flow path 38a was calculated by moving image analysis, and the spreadability of the blood on the reagent portion 42 was evaluated. The spreadability was evaluated by visual observation. The blood as a sample was prepared at Hct70, and a temperature was set to 5°C.

### [Result]

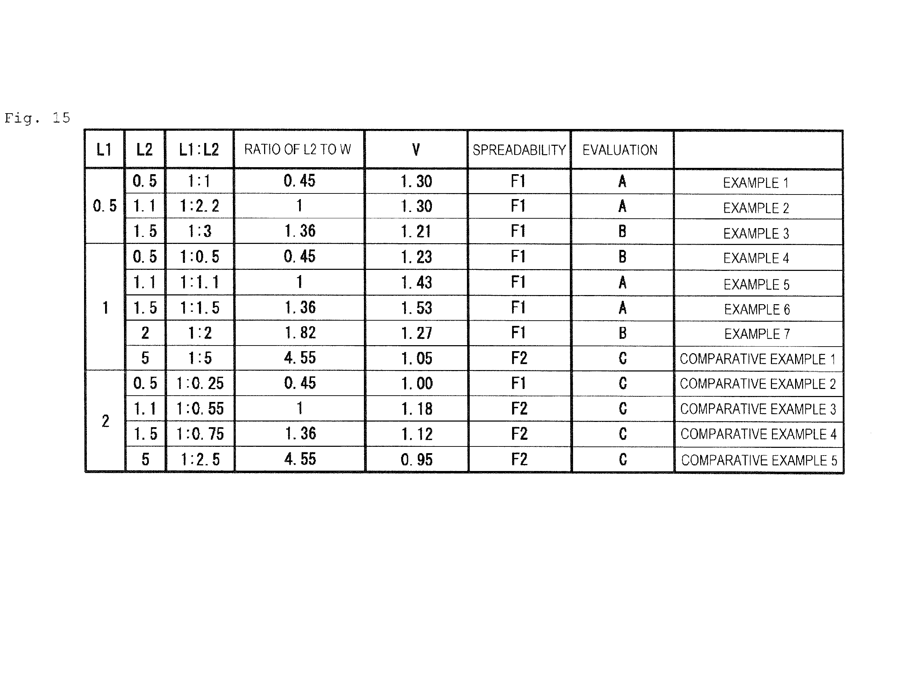

Test results of Examples 1 to 7 and Comparative Examples 1 to 5 are illustrated in Fig. 15. As illustrated in Fig. 15, in the evaluation of the test, those with the blood flow rate V of 1.3 mm/s or more were indicated by A, those with the blood flow rate V of 1.2 mm/s or more and less than 1.3 mm/s were indicated by B, and those with the blood flow rate V of less than 1.2 mm/s were indicated by C. Regarding the spreadability, those in which the spreadability of the blood on the reagent is good were indicated by F1, and those in which the spreadability of the blood on the reagent is not good were indicated by F2. More specifically, spreadability in which blood flows on the reagent portion 42 such that a flow end surface of the blood is substantially uniformly in the width direction was set to F1. Spreadability in which the flow end surface was a disordered shape when the blood flows on the reagent portion 42 was set to F2.

In Example 1, it is set that L1 = 0.5 mm, L2 = 0.5 mm, L1:L2 = 1:1, and L2/W = 0.45. In Example 1, V = 1.30 mm/s, and the spreadability was good, so that the evaluation was A. In Example 2, it is set that L1 = 0.5 mm, L2 = 1.1 mm, L1:L2 = 1:2.2, and L2/W = 1. In Example 2, V = 1.30 mm/s, and the spreadability was good, so that the evaluation was A. In Example 3, it is set L1 = 0.5 mm, L2 = 1.5 mm, L1:L2 = 1:3, and L2/W = 1.36. In Example 3, V = 1.21 mm/s, and the spreadability was good, so that the evaluation was B.

In Example 4, it is set that L1 = 1 mm, L2 = 0.5 mm, L1:L2 = 1:0.5, and L2/W = 0.45. In Example 4, V = 1.23 mm/s, and the spreadability was good, so that the evaluation was B. In Example 5, it is set that L1 = 1 mm, L2 = 1.1 mm, L1:L2 = 1:1.1, and L2/W = 1. In Example 5, V = 1.43 mm/s, and the spreadability was good, so that the evaluation was A. In Example 6, it is set that L1 = 1 mm, L2 = 1.5 mm, L1:L2 = 1:1.5, and L2/W = 1.36. In Example 6, V = 1.53 mm/s, and the spreadability was good, so that the evaluation was A. In Example 7, it is set that L1 = 1 mm, L2 = 2 mm, L1:L2 = 1:2, and L2/W = 1.82. In Example 7, V = 1.27 mm/s, and the spreadability was good, so that the evaluation was B.

In Comparative Example 1, it is set that L1 = 1 mm, L2 = 5 mm, L1:L2 = 1:5, and L2/W = 4.55. In Comparative Example 1, V = 1.05 mm/s, and the spreadability was not good, so that the evaluation was C. In all of Comparative Examples 2 to 5, L1 is set to 2 mm. In Comparative Example 2, the spreadability was good, whereas the blood flow rate V was 1.00 mm/s, so that the evaluation was C. In Comparative Examples 3 to 5, the blood flow rate V was less than 1.2 mm/s and the spreadability was not good, so that the evaluation was C.

As described above, in the test strip 12a, when the L2/W is 0.45 or more and 1.82 or less, a result that V is 1.2 mm/s or more and the spreadability is good was obtained. When the ratio of L2 to L1 is 0.5 or more and 3 or less, a result that V is 1.2 mm/s or more and the spreadability is good was obtained. Further, when L1 is 2 mm or more, a result that V is less than 1.2 mm/s was obtained. When the thickness of the flow path 38a was set to 130 µm, the relationship of L1:L2 was also the same as that when the flow path thickness was 90 µm. By setting L1 to 0.5 mm or more and 1 mm or less and L2 to 0.5 mm or more and 2 mm or less, more preferably 0.5 mm or more and 1.5 mm or less, even a small amount of sample of 1 µL or less can be efficiently drawn into the flow path 38a. In other words, because the intake portion 24a functions as a liquid pool at the first end of the test strip 12a, even a small amount of sample of 1 µL or less can be efficiently drawn into the flow path 38a. By setting L1 and L2 to such sizes, even a small amount of sample can be easily applied to an open surface side of the intake portion 24a. Also in the test strip 12a, one surface of the intake portion 24a in the arrow Z direction is covered with the fifth plate body 80E, whereas the other surface in the arrow Z direction is opened. When the entire surface of the first plate body 80A was the light shielding portion 32a, and the fifth plate body 80E and the sixth plate body 80F were formed of transparent members, the shape of the intake portion 24a is easily visible in the plan view of the test strip 12a from the Z direction, so that it is easy for the patient to apply the blood to the intake portion 24a.

The above embodiments are summarized as follows.

The above embodiment discloses a test strip (12, 12a) that includes a main body portion (20, 20a) to be attached to a component measurement device (14, 14a). The main body portion includes a light shielding portion (32), a reagent that reacts with a sample, and a flow path (38, 38a) that is provided in the test strip as a cavity (22, 22a) having an intake portion (24, 24a) at one end of the test strip and that introduces the sample to a measurement spot (44), and the light shielding portion defines the measurement spot.

In the test strip described above, the light shielding portion may be provided on a planar surface of the test strip, and may define at least both sides of the measurement spot in the width direction.

In the test strip described above, the light shielding portion may include an aperture (34, 86a) that defines the measurement spot, and a width dimension of the aperture along a width direction of the main body portion may be smaller than a width dimension of the flow path.

In the test strip described above, the aperture may be provided at a position overlapping, in a plan view, a reagent portion (42) carrying the reagent.

In the test strip described above, the light shielding portion may be formed of a black film member having a light shielding ratio of 90% or more.

In the test strip described above, the sample may be blood, and the reagent may be colored in accordance with an amount of glucose in the blood.

The above embodiment discloses a test strip that includes a main body portion to be attached to a component measurement device. The main body portion includes a light shielding layer (28A, 80A), a reagent that reacts with a sample, a spacer layer (28C, 28D, 80C, 80D) that has a flow path formed of a cavity that introduces the sample to a measurement spot, the flow path having an intake portion provided at one end of the test strip, and a first cover layer (28B, 80B) and a second cover layer (28E, 80E) that cover upper and lower surfaces of the spacer layer. The light shielding layer defines the measurement spot.

In the test strip described above, the flow path may be formed by covering the upper surface of the spacer layer with the first cover layer and covering the lower surface of the spacer layer with the second cover layer, and the intake portion may be formed by opening the one end of the flow path to an outside of the test strip.

In the test strip described above, the intake portion may be formed in a quadrangular shape in a plan view, and a ratio of a length (L2) of the intake portion along a minor axis direction of the main body portion to a length (L1) of the intake portion along a major axis direction of the main body portion may be 0.5 or more and 3 or less.

In the test strip described above, a ratio of the length of the intake portion along the minor axis direction of the main body portion to a width (W) of the flow path may be 0.45 or more and 1.82 or less.

In the test strip described above, the main body portion may be provided with an air vent portion (40, 40a), which is opened in at least one surface of the test strip and through which air from the flow path is vented, by stacking the light shielding layer, the spacer layer, the first cover layer, and the second cover layer.

In the test strip described above, the main body portion may include a third cover layer (80F) stacked on the second cover layer so as to cover at least a part of the air vent portion.

The above embodiment discloses a component measurement system (10) including a test strip that includes a main body portion for holding a sample, and a component measurement device to which the test strip is attached and that optically measures a component contained in the sample. The component measurement device is configured to emit measurement light traveling in a thickness direction of the main body portion, the main body portion includes a light shielding portion, and the light shielding portion has an aperture through which a measurement spot of the measurement light is formed on a reaction product of a reagent and the sample.

In the component measurement system described above, the aperture may be disposed inside an emitting-side passage opening (66a, 110b), through which the measurement light emitted from the component measurement device passes, in a state in which the test strip is attached to the component measurement device.

In the component measurement system described above, the aperture may be disposed inside a light receiving-side passage opening (66b, 110a) of a light receiving unit (74, 92) of the component measurement device in a state in which the test strip is attached to the component measurement device, so that the light receiving unit receives the measurement light transmitted through the main body portion.

## Claims

1. A test strip (12, 12a) comprising:
a main body portion (20, 20a) to be attached to a component measurement device (14, 14a), wherein
the main body portion (20, 20a) includes:
a reagent adapted to react with a sample, and
a flow path (38, 38a) that is provided in the test strip (12, 12a) as a cavity (22, 22a) having an intake portion (24, 24a) at one end of the test strip (12, 12a) and that is configured to introduce the sample to a measurement spot (44), and an aperture (34, 34a) that defines the measurement spot (44), and
a width dimension of the aperture (34, 34a) along a width direction of the main body portion (20, 20a) is smaller than a width dimension of the flow path (38, 38a),
**characterized in that**
a light shielding portion (32, 32a) having the aperture (34, 34a) is provided on the test strip (12, 12a) and arranged to shield at least a part of the measurement light emitted by the component measurement device (14,14a).

2. The test strip (12, 12a) according to claim 1, wherein
the light shielding portion (32, 32a) is located on a planar surface of the test strip (12, 12a), and defines at least both sides of the measurement spot (44) in a width direction.

3. The test strip (12, 12a) according to any one of claims 1 to 2, wherein
the aperture (34, 34a) is located at a position overlapping, in a plan view, a reagent portion (42) carrying the reagent.

4. The test strip (12, 12a) according to any one of claims 1 to 3, wherein
the light shielding portion (32, 32a) is formed of a black film member having a light shielding ratio of 90% or more.

5. The test strip (12, 12a) according to any one of claims 1 to 4 wherein
the sample is blood, and
the reagent is colored in accordance with an amount of glucose in the blood.

6. A test strip (12, 12a) according to claim 1 comprising:
a spacer layer (28C, 28D, 80C, 80D) that comprises said flow path (38, 38a), and
a first cover layer (28B, 80B) and a second cover layer (28E, 80E) that cover upper and lower surfaces of the spacer layer (28C, 28D, 80C, 80D).

7. The test strip (12, 12a) according to claim 6, wherein
the flow path (38, 38a) is formed by covering the upper surface of the spacer layer (28C, 28D, 80C, 80D) with the first cover layer (28B, 80B) and covering the lower surface of the spacer layer (28C, 28D, 80C, 80D) with the second cover layer (28E, 80E), and
the intake portion (24, 24a) is formed by opening the one end of the flow path (38, 38a) to an outside of the test strip (12, 12a).

8. The test strip (12, 12a) according to claim 7, wherein
the intake portion (24, 24a) has a quadrangular shape in a plan view, and
a ratio of a length (L2) of the intake portion (24, 24a) along a minor axis direction of the main body portion (20, 20a) to a length (L1) of the intake portion (20, 24a) along a major axis direction of the main body portion (20, 20a) is 0.5 or more and 3 or less.

9. The test strip (12, 12a) according to claim 7 or 8, wherein,
a ratio of the length (L2) of the intake portion (24, 24a) along the minor axis direction of the main body portion (20, 20a) to a width of the flow path (38, 38a) is 0.45 or more and 1.82 or less.

10. The test strip (12, 12a) according to any one of claims 6 to 9, wherein
the main body portion (20, 20a) is provided with an air vent portion (40, 40a) that is open in at least one surface of the test strip (12, 12a) and through that is configured to allow air from the flow path (38, 38a) to be vented, by stacking the light shielding layer (32, 32a), the spacer layer (28C, 28D, 80C, 80D), the first cover layer (28B, 80B), and the second cover layer (28E, 80E).

11. The test strip (12, 12a) according to claim 10, wherein
the main body portion (20, 20a) comprises a third cover layer (80F) stacked on the second cover layer (28E, 80E) so as to cover at least a part of the air vent portion (40, 40a).

12. A component measurement system (10) comprising:
a test strip (12, 12a) according to any of the previous claims, and
a component measurement device (14, 14a) to which the test strip (12, 12a) is attachable and that is configured to optically measure a component contained in the sample, wherein
the component measurement device (14, 14a) is configured to emit measurement light traveling in a thickness direction of the main body portion (20, 20a) of said test strip (12, 12a) and wherein a photo diode is included as a light receiving element (72) in a light receiving unit (74) in the component measurement device (14, 14a) to detect the measurement spot of a test strip (12, 12a) introduced into the component measurement device.

13. The component measurement system (10) according to claim 12, wherein
the aperture (34, 34a) of the test strip is configured to be disposable inside an emitting-side passage opening (66a, 110b) of the component measurement system (10), through which the measurement light emitted from the component measurement device (14, 14a) passes, in a state where the test strip (12, 12a) is attached to the component measurement device (14, 14a).

14. The component measurement system (10) according to claim 12 or 13, wherein
the aperture (34, 34a) is disposed inside a light receiving-side passage opening (66b, 110a) of a light receiving unit of the component measurement device (14, 14a) in a state where the test strip (12, 12a) is attached to the component measurement device (14, 14a), the light receiving unit being configured to receive the measurement light transmitted through the main body portion (20, 20a).

## Patentansprüche

1. Teststreifen (12, 12a), umfassend:
einen Hauptkörperabschnitt (20, 20a), der an einer Komponentenmessvorrichtung (14, 14a) anzubringen ist, wobei
der Hauptkörperabschnitt (20, 20a) umfasst:
ein Reagenz, das dazu ausgelegt ist, mit einer Probe zu reagieren, und
einen Strömungsweg (38, 38a), der in dem Teststreifen (12, 12a) als ein Hohlraum (22, 22a), der einen Aufnahmeabschnitt (24, 24a) an einem Ende des Teststreifens (12, 12a) aufweist, vorgesehen ist und der so konfiguriert ist, dass er die Probe zu einer Messstelle (44) einführt, und
eine Öffnung (34, 34a), welche die Messstelle (44) definiert, und
eine Breitenabmessung von der Öffnung (34, 34a) entlang einer Breitenrichtung des Hauptkörperabschnitts (20, 20a) kleiner ist als eine Breitenabmessung des Strömungswegs (38, 38a),
**dadurch gekennzeichnet, dass**
ein Lichtabschirmungsabschnitt (32, 32a), der die Öffnung (34, 34a) aufweist, auf dem Teststreifen (12, 12a) vorgesehen und so angeordnet ist, dass er mindestens einen Teil des von der Komponentenmessvorrichtung (14, 14a) emittierten Messlichts abschirmt.

2. Teststreifen (12, 12a) nach Anspruch 1, wobei
der Lichtabschirmungsabschnitt (32, 32a) auf einer ebenen Fläche des Teststreifens (12, 12a) angeordnet ist und mindestens beide Seiten der Messstelle (44) in einer Breitenrichtung definiert.

3. Teststreifen (12, 12a) nach einem der Ansprüche 1 bis 2, wobei
die Öffnung (34, 34a) an einer Position angeordnet ist, die in einer Draufsicht einen Reagenzabschnitt (42) überlappt, der das Reagenz trägt.

4. Teststreifen (12, 12a) nach einem der Ansprüche 1 bis 3, wobei
der Lichtabschirmungsabschnitt (32, 32a) aus einem schwarzen Filmelement gebildet ist, das ein Verhältnis der Lichtabschirmung von 90 % oder mehr aufweist.

5. Teststreifen (12, 12a) nach einem der Ansprüche 1 bis 4, wobei
die Probe Blut ist, und
das Reagenz entsprechend der Menge an Glukose im Blut gefärbt ist.

6. Teststreifen (12, 12a) nach Anspruch 1, umfassend:
eine Abstandsschicht (28C, 28D, 80C, 80D), die den Strömungsweg (38, 38a) umfasst, und
eine erste Deckschicht (28B, 80B) und eine zweite Deckschicht (28E, 80E), welche die Ober- und Unterseite der Abstandsschicht (28C, 28D, 80C, 80D) bedecken.

7. Teststreifen (12, 12a) nach Anspruch 6, wobei
der Strömungsweg (38, 38a) dadurch gebildet wird, indem die Oberseite der Abstandsschicht (28C, 28D, 80C, 80D) mit der ersten Deckschicht (28B, 80B) bedeckt wird und die Unterseite der Abstandsschicht (28C, 28D, 80C, 80D) mit der zweiten Deckschicht (28E, 80E) bedeckt wird, und
der Aufnahmeabschnitt (24, 24a) dadurch gebildet wird, indem das eine Ende des Strömungswegs (38, 38a) zu einer Außenseite des Teststreifens (12, 12a) hin geöffnet ist.

8. Teststreifen (12, 12a) nach Anspruch 7, wobei
der Aufnahmeabschnitt (24, 24a) in der Draufsicht eine viereckige Form aufweist, und
ein Verhältnis der Länge (L2) des Aufnahmeabschnitts (24, 24a) entlang einer Nebenachsenrichtung des Hauptkörperabschnitts (20, 20a) zu einer Länge (L1) des Aufnahmeabschnitts (20, 24a) entlang einer Hauptachsenrichtung des Hauptkörperabschnitts (20, 20a) 0,5 oder mehr und 3 oder weniger beträgt.

9. Teststreifen (12, 12a) nach Anspruch 7 oder 8, wobei,
ein Verhältnis der Länge (L2) des Aufnahmeabschnitts (24, 24a) entlang der Nebenachsenrichtung des Hauptkörperabschnitts (20, 20a) zu einer Breite des Strömungswegs (38, 38a) 0,45 oder mehr und 1,82 oder weniger beträgt.

10. Teststreifen (12, 12a) nach einem der Ansprüche 6 bis 9, wobei
der Hauptkörperabschnitt (20, 20a) mit einem Entlüftungsabschnitt (40, 40a) versehen ist, der in mindestens einer Oberfläche des Teststreifens (12, 12a) offen ist und der so konfiguriert ist, dass er es ermöglicht, Luft dadurch aus dem Strömungsweg (38, 38a) entweichen zu lassen, indem die Lichtabschirmschicht (32, 32a), die Abstandsschicht (28C, 28D, 80C, 80D), die erste Deckschicht (28B, 80B) und die zweite Deckschicht (28E, 80E) übereinander angeordnet sind.

11. Teststreifen (12, 12a) nach Anspruch 10, wobei
der Hauptkörperabschnitt (20, 20a) eine dritte Deckschicht (80F) umfasst, die auf der zweiten Deckschicht (28E, 80E) gestapelt ist, um mindestens einen Teil des Entlüftungsabschnitts (40, 40a) abzudecken.

12. Komponentenmesssystem (10) umfassend:
einen Teststreifen (12, 12a) nach einem der vorhergehenden Ansprüche, und
eine Komponentenmessvorrichtung (14, 14a), an welcher der Teststreifen (12, 12a) befestigt werden kann und die so konfiguriert ist, dass sie eine in der Probe enthaltene Komponente optisch misst, wobei
die Komponentenmessvorrichtung (14, 14a) so konfiguriert ist, dass sie Messlicht aussendet, das sich in einer Dickenrichtung des Hauptkörperabschnitts (20, 20a) von dem Teststreifen (12, 12a) bewegt, und wobei eine Fotodiode als ein Lichtempfangselement (72) in einer Lichtempfangseinheit (74) in der Komponentenmessvorrichtung (14, 14a) enthalten ist, um die Messstelle eines in die Komponentenmessvorrichtung eingeführten Teststreifens (12, 12a) zu erfassen.

13. Komponentenmesssystem (10) nach Anspruch 12, wobei
die Öffnung (34, 34a) des Teststreifens so konfiguriert ist, dass sie innerhalb einer Durchgangsöffnung (66a, 110b) der emittierenden Seite des Komponentenmesssystems (10), durch die das von der Komponentenmessvorrichtung (14, 14a) emittierte Messlicht hindurchtritt, in einem Zustand angeordnet werden kann, in welchem der Teststreifen (12, 12a) an der Komponentenmessvorrichtung (14, 14a) angebracht ist.

14. Komponentenmesssystem (10) nach Anspruch 12 oder 13, wobei
die Öffnung (34, 34a) innerhalb einer lichtempfangenden Seite der Durchgangsöffnung (66b, 110a) einer Lichtempfangseinheit der Komponentenmessvorrichtung (14, 14a) in einem Zustand angeordnet ist, in welchem der Teststreifen (12, 12a) an der Komponentenmessvorrichtung (14, 14a) angebracht ist, wobei die Lichtempfangseinheit so konfiguriert ist, dass sie das durch den Hauptkörperabschnitt (20, 20a) hindurchgelassene Messlicht empfängt.

## Revendications

1. Bandelette de test (12, 12a) comprenant :
une partie corps principal (20, 20a) destinée à être fixée à un dispositif de mesure de composant (14, 14a)
la partie corps principal (20, 20a) comprenant :
un réactif adapté pour réagir avec un échantillon, et
un trajet d'écoulement (38, 38a) étant prévu dans la bandelette de test (12, 12a) sous forme de cavité (22, 22a) ayant une partie admission (24, 24a) à une extrémité de la bandelette de test (12, 12a) et qui est configurée pour introduire l'échantillon dans un point de mesure (44), et
une ouverture (34, 34a) qui définit le point de mesure (44), et
une dimension de largeur de l'ouverture (34, 34a) le long d'une direction de largeur de la partie corps principal (20, 20a) étant inférieure à une dimension de largeur du trajet d'écoulement (38, 38a),
**caractérisée en ce**
**qu'**une partie de protection contre la lumière (32, 32a) présentant l'ouverture (34, 34a) est prévue sur la bandelette de test (12, 12a) et agencée pour protéger au moins une partie de la lumière de mesure émise par le dispositif de mesure de composant (14, 14a).

2. Bandelette de test (12, 12a) selon la revendication 1, dans laquelle
la partie de protection contre la lumière (32, 32a) est située sur une surface plane de la bandelette de test (12, 12a) et définit au moins les deux côtés du point de mesure (44) dans le sens de la largeur.

3. Bandelette de test (12, 12a) selon l'une quelconque des revendications 1 à 2, dans laquelle
l'ouverture (34, 34a) est située à une position chevauchant, dans une vue en plan, une partie réactif (42) comportant le réactif.

4. Bandelette de test (12, 12a) selon l'une quelconque des revendications 1 à 3, dans laquelle
la partie de protection contre la lumière (32, 32a) est formée d'un élément en film noir ayant un rapport de protection contre la lumière de 90 % ou plus.

5. Bandelette de test (12, 12a) selon l'une quelconque des revendications 1 à 4, dans laquelle
l'échantillon est du sang, et
le réactif est coloré en fonction d'une quantité de glucose dans le sang.

6. Bandelette de test (12, 12a) selon la revendication 1, comprenant :
une couche d'espacement (28C, 28D, 80C, 80D) qui comprend ledit trajet d'écoulement (38, 38a), et
une première couche de couverture (28B, 80B) et une deuxième couche de couverture (28E, 80E) qui recouvrent les surfaces supérieure et inférieure de la couche d'espacement (28C, 28D, 80C, 80D).

7. Bandelette de test (12, 12a) selon la revendication 6, dans laquelle
le trajet d'écoulement (38, 38a) est formé en recouvrant la surface supérieure de la couche d'espacement (28C, 28D, 80C, 80D) avec la première couche de couverture (28B, 80B) et en recouvrant la surface inférieure de la couche d'espacement (28C, 28D, 80C, 80D) avec la deuxième couche de couverture (28E, 80E), et
la partie admission (24, 24a) est formée par ouverture d'une extrémité du trajet d'écoulement (38, 38a) vers l'extérieur de la bandelette de test (12, 12a).

8. Bandelette de test (12, 12a) selon la revendication 7, dans laquelle
la partie admission (24, 24a) présente une forme quadrangulaire dans une vue en plan, et
un rapport entre une longueur (L2) de la partie admission (24, 24a) le long d'une direction d'axe secondaire de la partie corps principal (20, 20a) et une longueur (L1) de la partie admission (20, 24a) le long d'une direction d'axe principal de la partie corps principal (20, 20a) est de 0,5 ou plus et 3 ou moins.

9. Bandelette de test (12, 12a) selon la revendication 7 ou 8, dans laquelle,
un rapport de la longueur (L2) de la partie admission (24, 24a) le long de la direction d'axe secondaire de la partie corps principal (20, 20a) à une largeur du trajet d'écoulement (38, 38a) est de 0,45 ou plus et de 1,82 ou moins.

10. Bandelette de test (12, 12a) selon l'une quelconque des revendications 6 à 9, dans laquelle
la partie corps principal (20, 20a) est pourvue d'une partie évent d'air (40, 40a) qui est ouverte dans au moins une surface de la bandelette de test (12, 12a) et qui est configurée pour permettre à l'air provenant du trajet d'écoulement (38, 38a) d'être éventé, par empilement de la couche de protection contre la lumière (32, 32a), de la couche d'espacement (28C, 28D, 80C, 80D), de la première couche de couverture (28B, 80B) et de la deuxième couche de couverture (28E, 80E).

11. Bandelette de test (12, 12a) selon la revendication 10, dans laquelle
la partie corps principal (20, 20a) comprend une troisième couche de couverture (80F) empilée sur la deuxième couche de couverture (28E, 80E) de manière à recouvrir au moins une partie de la partie évent (40, 40a).

12. Système de mesure de composant (10) comprenant :
une bandelette de test (12, 12a) selon l'une quelconque des revendications précédentes, et
un dispositif de mesure de composant (14, 14a) auquel la bandelette de test (12, 12a) peut être fixée et qui est configuré pour mesurer optiquement un composant contenu dans l'échantillon,
le dispositif de mesure de composant (14, 14a) étant configuré pour émettre une lumière de mesure se déplaçant dans le sens de l'épaisseur de la partie corps principal (20, 20a) de ladite bandelette de test (12, 12a) et une photodiode étant incluse en tant qu'élément récepteur de lumière (72) dans une unité de réception de lumière (74) dans le dispositif de mesure de composant (14, 14a) pour détecter le point de mesure d'une bandelette de test (12, 12a) introduite dans le dispositif de mesure de composant.

13. Système de mesure de composant (10) selon la revendication 12, dans lequel
l'ouverture (34, 34a) de la bandelette de test est configurée pour être placée à l'intérieur d'une ouverture de passage côté émission (66a, 110b) du système de mesure de composant (10), à travers laquelle ouverture passe la lumière de mesure émise par le dispositif de mesure de composant (14, 14a), dans un état où la bandelette de test (12, 12a) est fixée au dispositif de mesure de composant (14, 14a).

14. Système de mesure de composant (10) selon la revendication 12 ou 13, dans lequel
l'ouverture (34, 34a) est disposée à l'intérieur d'une ouverture de passage côté réception de lumière (66b, 110a) d'une unité de réception de lumière du dispositif de mesure de composant (14, 14a) dans un état où la bandelette de test (12, 12a) est fixée au dispositif de mesure de composant (14, 14a), l'unité de réception de lumière étant configurée pour recevoir la lumière de mesure transmise à travers la partie corps principal (20, 20a).
